(19) Europäisches Patentamt
European Patent Office
Office européen des brevets

(11) EP 0 802 905 B1

(12) **EUROPÄISCHE PATENTSCHRIFT**

(45) Veröffentlichungstag und Bekanntmachung des Hinweises auf die Patenterteilung:
**09.09.1998 Patentblatt 1998/37**

(21) Anmeldenummer: **96900897.8**

(22) Anmeldetag: **04.01.1996**

(51) Int Cl.[6]: **C07D 213/61**, A01N 43/40, C07D 405/12

(86) Internationale Anmeldenummer:
**PCT/EP96/00007**

(87) Internationale Veröffentlichungsnummer:
**WO 96/21645 (18.07.1996 Gazette 1996/33)**

(54) **SUBSTITUIERTE 2-PHENYLPYRIDINE ALS HERBIZIDE**

SUBSTITUTED 2-PHENYLPYRIDINES AS HERBICIDES

2-PHENYLPYRIDINES SUBSTITUEES UTILISEES COMME HERBICIDES

(84) Benannte Vertragsstaaten:
**AT BE CH DE ES FR GB IT LI NL**

(30) Priorität: **13.01.1995 DE 19500758**

(43) Veröffentlichungstag der Anmeldung:
**29.10.1997 Patentblatt 1997/44**

(73) Patentinhaber: **BASF AKTIENGESELLSCHAFT**
**67056 Ludwigshafen (DE)**

(72) Erfinder:
- **SCHÄFER, Peter**
  **D-67308 Ottersheim (DE)**
- **HAMPRECHT, Gerhard**
  **D-69469 Weinheim (DE)**
- **HEISTRACHER, Elisabeth**
  **D-67061 Ludwigshafen (DE)**
- **KLINTZ, Ralf**
  **D-67269 Grünstadt (DE)**
- **KÖNIG, Hartmann**
  **D-69115 Heidelberg (DE)**
- **HARREUS, Albrecht**
  **D-67063 Ludwigshafen (DE)**
- **GÖTZ, Norbert**
  **D-67547 Worms (DE)**
- **WALTER, Helmut**
  **D-67283 Obrigheim (DE)**
- **WESTPHALEN, Karl-Otto**
  **D-67346 Speyer (DE)**
- **MISSLITZ, Ulf**
  **D-67433 Neustadt (DE)**

(56) Entgegenhaltungen:
**EP-A- 0 463 492**
**EP-A- 0 548 593**
**EP-A- 0 553 630**
**DE-A- 4 323 916**
**DE-A- 4 335 810**

- **CHEMICAL ABSTRACTS, vol. 114, no. 11, 18.März 1991 Columbus, Ohio, US; abstract no. 96724k, N.P. MEL'NIKOVA ET AL. 'Photodegradation of the herbicide Zellek.' Seite 243; & IZVESTIYA TIMIRYAZEVSKOI SEL'SKOKHOZYAISTVENNOI AKADEMII, Nr. 3, 1990 MOSCOW, Seiten 155-160,**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 802 905 B1

## Beschreibung

Die vorliegende Erfindung betrifft neue substituierte 2-Phenylpyridine der Formel I

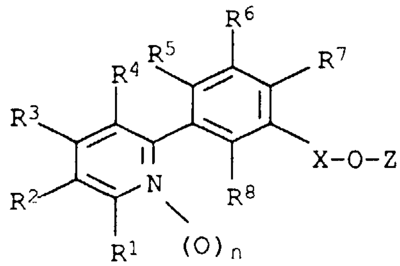

I

in der die Variablen folgende Bedeutungen haben:

| | |
|---|---|
| n | 0 oder 1; |
| $R^1$, $R^3$, $R^4$ | unabhängig voneinander Wasserstoff, Halogen, $C_1$-$C_4$-Alkyl, $C_1$-$C_4$-Halogenalkyl, Hydroxy, $C_1$-$C_4$-Alkoxy, $C_1$-$C_4$-Halogenalkoxy, Nitro, Amino, $C_1$-$C_4$-Alkylamino, Di-($C_1$-$C_4$-alkyl)-amino, Mercapto, $C_1$-$C_4$-Alkylthio, $C_1$-$C_4$-Halogenalkylthio, Cyano, Carboxyl, ($C_1$-$C_4$-Alkoxy)carbonyl, Aminocarbonyl, $C_1$-$C_4$-Alkylaminocarbonyl oder Di-($C_1$-$C_4$-alkyl)-aminocarbonyl; |
| $R^2$ | Wasserstoff, Halogen, $C_1$-$C_4$-Alkyl, $C_1$-$C_4$-Halogenalkyl, Cyano, Nitro, Amino, Hydroxy, $C_1$-$C_4$-Alkoxy, $C_1$-$C_4$-Halogenalkoxy, Mercapto, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl; |
| $R^5$ | Wasserstoff, Halogen oder Cyano; |
| $R^6$ und $R^8$ | unabhängig voneinander Wasserstoff oder Halogen; |
| $R^7$ | Wasserstoff, Cyano, Nitro, Hydroxy, Trifluormethylsulfonyloxy, Halogen, $C_1$-$C_4$-Alkyl, $C_1$-$C_4$-Halogenalkyl, $C_1$-$C_4$-Alkoxy oder $C_1$-$C_4$-Halogenalkoxy; |
| X | eine der folgenden Brücken: -CO-, -O-C($R^9$,$R^{10}$)-CO-, -S-C($R^9$,$R^{10}$)-CO-, -$CH_2$-CH($R^{11}$)-CO- oder -CH=C($R^{11}$)-CO-, wobei $R^9$ und $R^{10}$ unabhängig voneinander für Wasserstoff oder $C_1$-$C_4$-Alkyl und $R^{11}$ für Wasserstoff, Halogen oder $C_1$-$C_4$-Alkyl stehen; |
| Z | eine Gruppe |

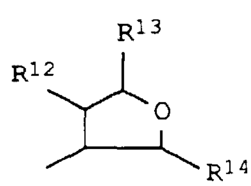

oder

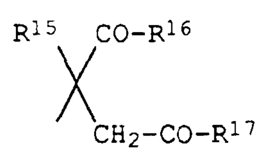

,

wobei

| | |
|---|---|
| $R^{12}$ | für Wasserstoff, Hydroxy, Halogen, $C_1$-$C_4$-Alkyl, $C_1$-$C_4$-Alkoxy, ($C_1$-$C_4$-Alkyl)carbonyloxy, ($C_1$-$C_4$-Halogenalkyl)carbonyloxy, ($C_3$-$C_6$-Cycloalkyl)carbonyloxy, Benzoyloxy, Benzylcarbonyloxy, Benzyloxy, ($C_1$-$C_4$-Alkoxy)carbonyloxy, ($C_1$-$C_4$-Alkylamino)carbonyloxy, Di-($C_1$-$C_4$-alkyl)-aminocarbonyloxy oder $C_1$-$C_3$-Alkylsulfonyloxy; |

$R^{13}$ und $R^{14}$ unabhängig voneinander für Wasserstoff, Hydroxy, $C_1$-$C_4$-Alkyl, $C_1$-$C_4$-Alkoxy oder ($C_1$-$C_4$-Alkyl)carbonyloxy;

$R^{15}$ für Wasserstoff oder $C_1$-$C_4$-Alkyl und

$R^{16}$ und $R^{17}$ unabhängig voneinander für Hydroxy, $C_1$-$C_6$-Alkoxy, $C_3$-$C_6$-Alkenyloxy, $C_3$-$C_6$-Alkinyloxy, $C_1$-$C_6$-Alkoxy-$C_2$-$C_4$-alkoxy, $C_3$-$C_6$-Cycloalkyloxy, Amino, $C_1$-$C_4$-Alkylamino, Di-($C_1$-$C_4$-alkyl)-amino, N-Pyrrolidinyl, N-Piperidinyl, N-Morpholinyl, $C_3$-$C_4$-Cycloalkylamino, Di-($C_3$-$C_6$-cycloalkyl)-amino, $C_3$-$C_6$-Alkenylamino oder $C_3$-$C_6$-Alkinylamino oder

$R^{16}$ und $R^{17}$ zusammen für eine Brücke -N($R^{18}$)-N($R^{19}$)- oder -N($R^{20}$)-stehen, wobei $R^{18}$ und $R^{19}$ jeweils Wasserstoff oder $C_1$-$C_4$-Alkyl und

$R^{20}$ Wasserstoff, $C_1$-$C_6$-Alkyl, $C_3$-$C_6$-Alkenyl, $C_3$-$C_6$-Alkinyl, $C_1$-$C_6$-Alkoxy oder $C_3$-$C_6$-Alkenyloxy

bedeuten,
sowie die landwirtschaftlich brauchbaren Salze der Verbindungen I, sofern diese existieren.

Außerdem betrifft die Erfindung

- die Verwendung der Verbindungen I als Herbizide und/oder zur Desikkation und/oder Defoliation von Pflanzen,
- herbizide Mittel und Mittel zur Desikkation und/oder Defoliation von Pflanzen, welche die Verbindungen I als wirksame Substanzen enthalten,
- Verfahren zur Bekämpfung von unerwünschtem Pflanzenwuchs und zur Desikkation und/oder Defoliation von Pflanzen mit den Verbindungen I,
- Verfahren zur Herstellung der Verbindungen I und von herbiziden Mitteln und Mitteln zur Desikkation und/oder Defoliation von Pflanzen unter Verwendung der Verbindungen I, sowie
- Zwischenprodukte der Formel III und ein Verfahren zu deren Herstellung.

In der älteren deutschen Anmeldung DE-A 43 23 916 (Veröffentlichungsdatum 19.01.95 : Stand der Technik im Sinne des Artikels 54, Absatz 3) werden u.a. 2-Phenylpyridine vom Typ der Verbindungen I als Herbizide und als desikkant/defoliant wirksame Verbindungen beschrieben. Bei geeigneter Wahl der Substituenten ergeben sich u.a. Verbindungen der Formel IIa

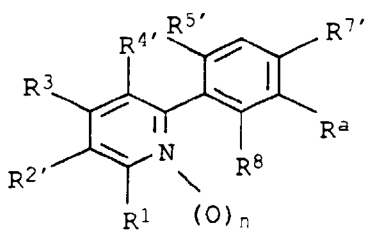

IIa

wobei

$R^{2'}$ für Halogen, Cyano, Nitro, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylthio oder $C_1$-$C_4$-Halogenalkylthio;
$R^{4'}$ für Wasserstoff, Nitro, Amino, Cyano, Hydroxy, Mercapto, Hydroxycarbonyl, Halogen, $C_1$-$C_4$-Alkyl, $C_1$-$C_4$-Halogenalkyl, $C_1$-$C_4$-Alkoxy, $C_1$-$C_4$-Halogenalkoxy, $C_1$-$C_4$-Alkylamino, Di-($C_1$-$C_4$-alkyl)-amino, $C_1$-$C_4$-Alkylthio, $C_1$-$C_4$-Halogenalkylthio oder ($C_1$-$C_4$-Alkoxy)carbonyl;
$R^{5'}$ für Wasserstoff oder Halogen;
$R^{7'}$ für Cyano, Nitro, Hydroxyl, Halogen oder Trifluormethyl;
$R^a$ für Alkoxycarbonyl-alkyl-oxy oder für verschiedene organische Reste, die u.a. über -CO-, -$CH_2$-CH(Halogen)-CO- oder -C=C($R^{11}$)-CO- gebunden sein können,

stehen.

Außerdem wird in der EP-A 548 593 gelehrt, daß Verbindungen der Formel IIb

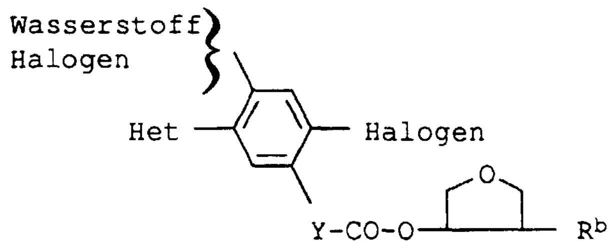

IIb

wobei Het für verschiedene Heterocyclen und Y u.a. für Bindung, $-OCH_2-$, -OCH ($C_{1-4}$-Alkyl)-, $-SCH_2-$ oder -S-CH ($C_{1-4}$-Alkyl) stehen und $R^b$ H, OH, Halogen, $C_{1-4}$-Alkoxy, Benzyloxy, $C_{1-3}$-Alkylsulfonyloxy oder bestimmte Reste -O-CO-R bedeutet, herbizid wirksam sind.

Des weiteren ist aus der DE-A 43 35 810 bekannt, daß 3-Phenyluracil-Derivate, bei denen der Phenylring in meta-Position zum Uracilteil eine Gruppe $-CO-O-C(R^{15},COR^c)-CH_2-COR^d$ trägt, wobei $COR^c$ und $COR^d$ Ester-, Thioester- oder Säureamidreste darstellen oder $R^c$ und $R^d$ zusammen eine Stickstoff- oder Distickstoffbrücke bilden, ebenfalls herbizid wirksam sind.

Die herbizide Wirkung der bekannten Verbindungen bezüglich der Schadpflanzen ist jedoch nicht immer voll befriedigend. Aufgabe der vorliegenden Erfindung war es demnach, neue herbizid wirksame Verbindungen bereitzustellen, mit denen sich unerwünschte Pflanzen besser als bisher gezielt bekämpfen lassen. Die Aufgabe erstreckt sich auch auf die Bereitstellung neuer desikkant/defoliant wirksamer Verbindungen.

Demgemäß wurden die eingangs definierten substituierten 2-Phenylpyridine der Formel I mit herbizider Wirkung sowie neue Zwischenprodukte III zu deren Herstellung gefunden.

Ferner wurden herbizide Mittel gefunden, die die Verbindungen I enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen I gefunden.

Des weiteren wurde gefunden, daß die Verbindungen I auch zur Defoliation und Desikkation von Pflanzenteilen geeignet sind, wofür Kulturpflanzen wie Baumwolle, Kartoffel, Raps, Sonnenblume, Sojabohne oder Ackerbohnen, insbesondere Baumwolle, in Betracht kommen. Diesbezüglich wurden Mittel zur Desikkation und/oder Defoliation von Pflanzen, Verfahren zur Herstellung dieser Mittel und Verfahren zur Desiccation und/oder Defoliation von Pflanzen mit den Verbindungen I gefunden.

Die Verbindungen der Formel I können je nach Substitutionsmuster ein oder mehrere Chiralitätszentren enthalten und liegen dann als Enantiomeren- oder Diastereomerengemische vor. Gegenstand der Erfindung sind sowohl die reinen Enantiomeren oder Diastereomeren als auch deren Gemische.

Die substituierten 2-Phenylpyridine I können in Form ihrer landwirtschaftlich brauchbaren Salze vorliegen, wobei es auf die Art des Salzes in der Regel nicht ankommt. Im allgemeinen kommen die Salze von solchen Basen und diejenigen Säureadditionssalze in Betracht, bei denen die herbizide Wirkung im Vergleich zu der freien Verbindung I nicht negativ beeinträchtigt ist.

Als Salze eignen sich besonders diejenigen der Alkalimetalle, vorzugsweise Natrium- und Kaliumsalze, der Erdalkalimetalle, vorzugsweise Calcium- und Magnesiumsalze, die der Übergangsmetalle, vorzugsweise Zink- und Eisensalze, sowie Ammoniumsalze, bei denen das Ammoniumion gewünschtenfalls ein bis vier $C_1$-$C_4$-Alkyl-, Hydroxy-$C_1$-$C_4$-alkylsubstituenten und/oder einen Phenyl- oder Benzylsubstituenten tragen kann, vorzugsweise Diisopropylammonium-, Tetramethylammonium-, Tetrabutylammonium-, Trimethylbenzylammonium- und Trimethyl-(2-hydroxyethyl)-ammoniumsalze, des weiteren Phosphoniumsalze, Sulfoniumsalze wie vorzugsweise Tri-($C_1$-$C_4$-alkyl)sulfoniumsalze, und Sulfoxoniumsalze wie vorzugseise Tri-($C_1$-$C_4$-alkyl)sulfoxoniumsalze.

Unter den Säureadditionssalzen sind in erster Linie die Hydrochloride und -bromide, Sulfate, Nitrate, Phosphate, Oxalate und die Dodecylbenzolsulfonate zu nennen.

Die bei der Definition der Substituenten $R^1$ bis $R^{20}$ verwendeten Bezeichnungen Alkyl, Halogenalkyl, Alkoxy, Halogenalkoxy, Alkoxyalkoxy, Alkylthio, Halogenalkylthio, Alkylamino, Dialkylamino, Cycloalkyloxy, Cycloalkylamino, Dicycloalkylamino, Alkoxycarbonyl, Alkoxycarbonyloxy, Alkylcarbonyloxy, Halogenalkylcarbonyloxy, Cycloalkylcarbonyloxy, Alkylaminocarbonyl, Dialkylaminocarbonyl, Alkylaminocarbonyloxy, Dialkylaminocarbonyloxy, Alkylsulfinyl, Halogenalkylsulfinyl, Alkylsulfonyl, Halogenalkylsulfonyl, Alkylsulfonyloxy, Alkenyl, Alkinyl, Alkenyloxy, Alkinyloxy, Alkenylamino und Alkinylamino stellen - wie die Bedeutung Halogen - Sammelbegriffe für individuelle Aufzählungen der einzelnen Gruppenmitglieder dar. Sämtliche Kohlenstoffketten, also alle Alkyl-, Halogenalkyl-, Alkenyl- und Alkinyl-Teile können geradkettig oder verzweigt sein. Halogenierte Substituenten tragen vorzugsweise ein bis fünf gleiche oder verschiedene Halogenatome.

Im einzelnen stehen beispielsweise:

- Halogen für: Fluor, Chlor, Brom oder Iod;

- $C_1$-$C_4$-Alkyl sowie der Alkyl-Teil von ($C_1$-$C_4$-Alkyl)carbonyloxy für: Methyl, Ethyl, n-Propyl, 1-Methylethyl, n-Butyl, 1-Methylpropyl, 2-Methylpropyl oder 1,1-Dimethylethyl;

- $C_1$-$C_4$-Halogenalkyl sowie der Halogenalkyl-Teil von ($C_1$-$C_4$-Halogenalkyl)carbonyloxy für: einen $C_1$-$C_4$-Alkylrest wie vorstehend genannt, der partiell oder vollständig durch Fluor, Chlor, Brom und/oder Iod substituiert ist, also z. B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Petafluorethyl, 2-Fluorpropyl,3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;

- $C_1$-$C_4$-Alkoxy sowie der Alkoxy-Teil von ($C_1$-$C_4$-Alkoxy)carbonyloxy für: Methoxy, Ethoxy, n-Propoxy, 1-Methylethoxy, n-Butoxy, 1-Methylpropoxy, 2-Methylpropoxy oder 1,1-Dimethylethoxy;

- $C_1$-$C_6$-Alkoxy für: $C_1$-$C_4$-Alkoxy wie vorstehend genannt, sowie für n-Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, n-Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;

- $C_1$-$C_4$-Halogenalkoxy für: $C_1$-$C_4$-Alkoxy wie vorstehend genannt, das partiell oder vollständig durch Fluor, Chlor, Brom und/ oder Iod substituiert ist, also z.B. Difluormethoxy, Trifluormethoxy, Chlordifluormethoxy, Bromdifluormethoxy, 2-Fluorethoxy, 2-Chlorethoxy, 2-Bromethoxy, 2-Iodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Fluorpropoxy, 2-Chlorporpoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorporpoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy oder Nonafluorbutoxy;

- $C_1$-$C_6$-Alkoxy-$C_2$-$C_4$-alkoxy für: durch $C_1$-$C_6$-Alkoxy wie vorstehend genannt substituiertes $C_2$-$C_6$-alkoxy, also z. B. für 2-(Methoxy)ethoxy, 2-(Ethoxy)ethoxy, 2-(n-Propoxy)ethoxy, 2-(1-Methylethoxy)ethoxy, 2-(n-Butoxy)ethoxy, 2-(1-Methylpropoxy)ethoxy, 2-(2-Methylpropoxy)ethoxy, 2-(1,1-Dimethylethoxy)ethoxy, 2-(Methoxy)propoxy, 2-(Ethoxy)propoxy, 2-(n-Propoxy)propoxy, 2-(1-Methylethoxy)propoxy, 2-(n-Butoxy)propoxy, 2-(1-Methylpropoxy) propoxy, 2-(2-Methylpropoxy)propoxy, 2-(1,1-Dimethylethoxy)propoxy, 2-(n-Pentoxy)propoxy, 2-(n-Hexoxy)propoxy, 3-(Methoxy)propoxy, 3-(Ethoxy)propoxy, 3-(n-Propoxy)propoxy, 3-(1-Methylethoxy)propoxy, 3-(n-Butoxy) propoxy, 3-(1-Methylpropoxy)propoxy, 3-(2-Methylpropoxy)propoxy, 3-(1,1-Dimethylethoxy)propoxy, 3-(n-Pentoxy)propoxy, 3-(n-Hexoxy)propoxy, 2-(Methoxy)butoxy, 2-(Ethoxy)butoxy, 2-(n-Propoxy)butoxy, 2-(1-Methylethoxy)butoxy, 2-(n-Butoxy)butoxy, 2-(1-Methylpropoxy)butoxy, 2-(2-Methylpropoxy)butoxy, 2-(1,1-Dimethylethoxy)butoxy, 2-(n-Pentoxy)butpoxy, 2-(n-Hexoxy)butoxy, 3-(Methoxy)butoxy, 3-(Ethoxy)butoxy, 3-(n-Propoxy)butoxy, 3-(1-Methylethoxy)butoxy, 3-(n-Butoxy)butoxy, 3-(1-Methylpropoxy)butoxy, 3-(2-Methylpropoxy)butoxy, 3-(1,1-Dimethylethoxy)butoxy, 4-(Methoxy)butoxy, 4-(Ethoxy)butoxy, 4-(n-Propoxy)butoxy, 4-(1-Methylethoxy)butoxy, 4-(n-Butoxy)butoxy, 4-(1-Methylpropoxy)butoxy, 4-(2-Methylpropoxy)butoxy, 4-(1,1-Dimethylethoxy)butoxy, 2-(n-Pentoxy)butoxy oder 2-(n-Hexoxy)butoxy;

- $C_1$-$C_4$-Alkylthio für: Methylthio, Ethylthio, n-Propylthio, 1-Methylethylthio, n-Butylthio, 1-Methylpropylthio, 2-Methylpropylthio oder 1,1-Dimethylethylthio;

- $C_1$-$C_4$-Halogenalkylthio für: $C_1$-$C_4$-Alkylthio wie vorstehend genannt, das partiell oder vollständig durch Fluor, Chlor, Brom und/oder Iod substituiert ist, also z.B. Difluormethylthio, Trifluormethylthio, Chlordifluormethylthio, Bromdifluormethylthio, 2-Fluorethylthio, 2-Chlorethylthio, 2-Bromethylthio, 2-Iodethylthio, 2,2-Difluorethylthio, 2,2,2-Trifluorethylthio, 2,2,2-Trichlorethylthio, 2-Chlor-2-fluorethylthio, 2-Chlor-2,2-difluorethylthio, 2,2,-Dichlor-2-fluorethylthio, Pentafluorethylthio, 2-Fluorpropylthio, 3-Fluorpropylthio, 2-Chlorpropylthio, 3-Chlorpropylthio,

2-Brompropylthio, 3-Brompropylthio, 2,2,-Difluorpropylthio, 2,3-Difluorpropylthio, 2,3-Dichlorpropylthio, 3,3,3-Trifluorpropylthio, 3,3,3-Trichlorpropylthio, 2,2,3,3,3-Pentafluorpropylthio, Heptafluorpropylthio, 1-(Fluormethyl)-2-fluorethylthio, 1-(Chlormethyl)-2-chlorethylthio, 1-(Brommethyl)-2-bromethylthio, 4-Fluorbutylthio, 4-Chlorbutylthio, 4-Brombutylthio oder Nonafluorbutylthio;

- $C_1$-$C_4$-Alkylamino sowie die Alkylamino-Teile von ($C_1$-$C_4$-Alkylamino)carbonyl und $C_1$-$C_4$-Alkylaminocarbonyloxy für: Methylamino, Ethylamino, n-Propylamino, 1-Methylethylamino, n-Butylamino, 1-Methyl-propylamino, 2-Methylpropylamino oder 1,1-Dimethylethylamino;

- Di-($C_1$-$C_4$-alkyl)-amino sowie die Dialkylamino-Teile von Di-($C_1$-$C_4$-alkyl)-aminocarbonyl und Di-($C_1$-$C_4$-alkyl)-aminocarbonyloxy für: z. B. N,N-Dimethylamino, N,N-Diethylamino, N,N-Diisopropylamino, N,N-Dibutylamino, N,N-Di-(1-methylpropyl)amino, N,N-Di-(2-methylpropyl)amino, N,N-Di-(1,1-dimethylethyl)amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylproyl)amino, N-(1,1-Dimethylethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)amino, N-Ethyl-N-(1,1-dimethylethyl)amino, N-(1-Methyletyl)-N-propylamino, N-Butyl-N-propylamino, N-(1-Methylpropyl)-N-propylamino, N-(2-Methylpropyl)-N-propylamino, N-(1,1-Dimethylethyl)-N-propylamino, N-Butyl-N-(1-methylethyl)amino, N-(1-Methylethyl)-N-(1-methylpropyl)amino, N-(1-Methylethyl)-N-(2-methylpropyl) amino, N-(1,1,-Dimethylethyl)-N-(1-methylethyl)amino, N-Butyl-N-(1-methylpropyl)amino-, N-Butyl-N-(2-methylpropyl)amino, N-Butyl-N-(1,1-dimethylethyl)amino, N-(1-Methylpropyl)-N-(2-methylpropyl)amino, N-(1,1-Dimethylethyl)-N-(1-methylpropyl)amino oder N-(1,1-Dimethylethyl)-N-(2-methylpropyl)amino;

- $C_1$-$C_4$-Alkylsulfinyl für: Methylsulfinyl, Ethylsulfinyl, n-Propylsulfinyl, 1-Methylethylsulfinyl, n-Butylsulfinyl, l-Methylpropylsulfinyl, 2-Methylpropylsulfinyl oder 1,1-Dimethylethylsulfinyl;

- $C_1$-$C_4$-Halogenalkylsulfinyl für: $C_1$-$C_4$-Alkylsulfinyl wie vorstehend genannt, das partiell oder vollständig durch Fluor, Chlor, Brom und/oder Iod substituiert ist, also z.B. Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-Iodethylsulfinyl, 2,2-Difluorethylsulfinyl, 2,2,2-Trifluorethylsulfinyl, 2,2,2-Trichlorethylsulfinyl, 2-Chlor-2-fluorethylsulfinyl, 2-Chlor-2,2-difluorethylsulfinyl, 2,2,-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropylsulfinyl, 2-Brompropylsulfinyl, 3-Brompropylsulfinyl, 2,2,-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl oder Nonafluorbutylsulfinyl;

- $C_1$-$C_4$-Alkylsulfonyl für: Methylsulfonyl, Ethylsulfonyl, n-Propylsulfonyl, 1-Methylethylsulfonyl, n-Butylsulfonyl, 1-Methylpropylsulfonyl, 2-Methylpropylsulfonyl oder 1,1-Dimethylethylsulfonyl;

- $C_1$-$C_4$-Halogenalkylsulfonyl für: $C_1$-$C_4$-Alkylsulfonyl wie vorstehend genannt, das partiell oder vollständig durch Fluor, Chlor, Brom und/oder Iod substituiert ist, also z.B. Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-Iodethylsulfonyl, 2,2-Difluorethylsulfonyl, 2,2,2-Trifluorethylsulfonyl, 2,2,2-Trichlorethylsulfonyl, 2-Chlor-2-fluorethylsulfonyl, 2-Chlor-2,2-difluorethylsulfonyl, 2,2,-Dichlor-2-fluorethylsulfonyl, Pentafluorethylsulfonyl, 2-Fluorpropylsulfonyl, 3-Fluorpropylsulfonyl, 2-Chlorpropylsulfonyl, 3-Chlorpropylsulfonyl, 2-Brompropylsulfonyl, 3-Brompropylsulfonyl, 2,2,-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluorpropylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl oder Nonafluorbutylsulfonyl;

- $C_1$-$C_3$-Alkylsulfonyloxy für: Methylsulfonyloxy, Ethylsulfonyloxy, n-Propylsulfonyloxy oder 1-Methylethylsulfonyloxy;

- die Cycloalkylteile von ($C_3$-$C_6$-Cycloalkyl)carbonyloxy, $C_3$-$C_6$-Cycloalkylamino und Di-($C_3$-$C_6$-cycloalkyl)-amino für: Cyclopropyl, Cyclobutyl, Cyclopentyl oder Cyclohexyl;

- $C_3$-$C_6$-Alkenyl sowie die Alkenylteile von $C_3$-$C_6$-Alkenyloxy und $C_3$-$C_6$-Alkenylamino für: Prop-1-en-1-yl, Prop-2-en-1-yl, 1-Methylethenyl, n-Buten-1-yl, n-Buten-2-yl, n-Buten-3-yl, 1-Methyl-prop-1-en-1-yl, 2-Methyl-prop-1-en-1-yl, 1-Methylprop-2-en-1-yl, 2-Methyl-prop-2-en-1-yl, n-Penten-1-yl, n-Penten-2-yl, n-Penten-3-yl, n-Penten-4-yl, 1-Methyl-but-1-en-1-yl, 2-Methyl-but-1-en-1-yl, 3-Methyl-but-1-en-1-yl, 1-Methyl-but-2-en-1-yl, 2-Methyl-but-2-en-1-yl, 3-Methyl-but-2-en-1-yl, 1-Methyl-but-3-en-1-yl, 2-Methyl-but-3-en-1-yl, 3-Methyl-but-3-en-1-yl, 1,1-Dimethyl-prop-2-en-1-yl, 1,2-Dimethyl-prop-1-en-1-yl, 1,2-Dimethyl-prop-2-en-1-yl, 1-Ethyl-prop-1-en-2-yl, 1-Ethyl-prop-2-en-1-yl, n-Hex-1-en-1-yl, n-Hex-2-en-1-yl, n-Hex-3-en-1-yl, n-Hex-4-en-1-yl, n-Hex-5-en-1-yl, 1-Methyl-pent-1-en-1-yl, 2-Methyl-pent-1-en-1-yl, 3-Methyl-pent-1-en-1-yl, 4-Methyl-pent-1-en-1-yl, 1-Methyl-pent-2-en-1-yl, 2-Methyl-pent-2-en-1-yl, 3-Methyl-pent-2-en-1-yl, 4-Methylpent-2-en-1-yl, l-Methyl-pent-3-en-1-yl, 2-Methyl-pent-3-en-1-yl, 3-Methyl-pent-3-en-1-yl, 4-Methyl-pent-3-en-1-yl, 1-Methyl-pent-4-en-1-yl, 2-Methyl-pent-4-en-1-yl, 3-Methyl-pent-4-en-1-yl, 4-Methyl-pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethylbut-3-en-1-yl, 3,3-Dimethyl-but-1-en-1-yl, 3,3-Dimethyl-but-2-en-1-yl, 1-Ethyl-but-1-en-1-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-l-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;

- $C_3$-$C_6$-Alkinyl sowie die Alkinylteile von $C_3$-$C_6$-Alkinyloxy und $C_3$-$C_6$-Alkinylamino für: Prop-1-in-1-yl, Prop-2-in-1-yl, n-But-1-in-1-yl, n-But-1-in-3-yl, n-But-1-in-4-yl, n-But-2-in-1-yl, n-Pent-1-in-1-yl, n-Pent-1-in-3-yl, n-Pent-1-in-4-yl, n-Pent-1-in-5-yl, n-Pent-2-in-1-yl, n-Pent-2-in-4-yl, n-Pent-2-in-5-yl, 3-Methyl-but-1-in-3-yl, 3-Methyl-but-1-in-4-yl, n-Hex-1-in-1-yl, n-Hex-1-in-3-yl, n-Hex-1-in-4-yl, n-Hex-1-in-5-yl, n-Hex-1-in-6-yl, n-Hex-2-in-1-yl, n-Hex-2-in-4-yl, n-Hex-2-in-5-yl, n-Hex-2-in-6-yl, n-Hex-3-in-1-yl, n-Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl.

Im Hinblick auf die Verwendung der erfindungsgemäßen Verbindungen der Formel I als Herbizide und/oder als defoliant/desikkant wirksame Verbindungen haben die Variablen vorzugsweise folgende Bedeutungen, und zwar jeweils für sich allein oder in Kombination:

| | |
|---|---|
| n | Null; |
| $R^1$, $R^3$, $R^4$, $R^5$ | unabhängig voneinander Wasserstoff oder Halogen; |
| $R^2$ | Halogen oder $C_1$-$C_4$-Halogenalkyl mit ein bis fünf Halogenatomen; |
| $R^6$, $R^8$ | Wasserstoff; |
| $R^7$ | Cyano oder Halogen; |
| $R^9$, $R^{10}$ | unabhängig voneinander Wasserstoff oder $C_1$-$C_4$-Alkyl; $R^{11}$ Wasserstoff oder Halogen; |
| $R^{12}$,$R^{13}$, $R^{14}$ | unabhängig voneinander Wasserstoff, Hydroxy, $C_1$-$C_4$-Alkyl oder ($C_1$-$C_4$-Alkyl)carbonyloxy; |
| $R^{15}$ | Wasserstoff; |
| $R^{16}$, $R^{17}$ | unabhängig voneinander $C_1$-$C_6$-Alkoxy, $C_3$-$C_6$-Alkenyloxy, Amino, $C_1$-$C_4$-Alkylamino, Di-($C_1$-$C_4$-alkyl)-amino oder zusammen eine Brücke -NH-NH-, -N($C_1$-$C_4$-Alkyl)-N($C_1$-$C_4$-Alkyl)- oder -N($R^{20}$)-, wobei |
| $R^{20}$ | für Wasserstoff oder $C_1$-$C_4$-Alkyl steht. |

Besonders bevorzugt sind die in der folgenden Tabelle 1 aufgeführten Verbindungen Ia (≙ I mit n = Null; $R^1$, $R^3$, $R^6$ und $R^8$ = Wasserstoff) :

Tabelle 1

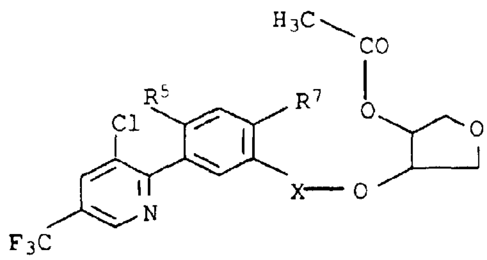

Ia

| Nr. | $R^5$ | $R^7$ | X | Schmp. [°C]; IR-Daten ($\gamma$[$cm^{-1}$]); $^1$H-NMR-Daten ($CDCl_3$/TMS, $\delta$ [ppm]) |
|---|---|---|---|---|
| Ia.01 | H | Cl | -CO- | |
| Ia.02 | F | Cl | -CO- | |
| Ia.03 | H | CN | -CO- | |
| Ia.04 | F | CN | -CO- | |
| Ia.05 | H | Cl | $-O-CH_2-CO-$ | |
| Ia.06 | F | Cl | $-O-CH_2-CO-$ | |
| Ia.07 | H | CN | $-O-CH_2-CO-$ | |
| Ia.08 | F | CN | $-O-CH_2-CO-$ | |
| Ia.09 | H | Cl | $-O-CH(CH_3)-CO-$ | |
| Ia.10 | F | Cl | $-O-CH(CH_3)-CO-$ | siehe Herstellungsbeispiel |
| Ia.11 | H | CN | $-O-CH(CH_3)-CO-$ | |
| Ia.12 | F | CN | $-O-CH(CH_3)-CO-$ | |
| Ia.13 | H | Cl | $-S-CH_2-CO-$ | |
| Ia.14 | F | Cl | $-S-CH_2-CO-$ | |
| Ia.15 | H | CN | $-S-CH_2-CO-$ | |
| Ia.16 | F | CN | $-S-CH_2-CO-$ | |
| Ia.17 | H | Cl | $-S-CH(CH_3)-CO-$ | |
| Ia.18 | F | Cl | $-S-CH(CH_3)-CO-$ | |
| Ia.19 | H | CN | $-S-CH(CH_3)-CO-$ | |
| Ia.20 | F | CN | $-S-CH(CH_3)-CO-$ | |
| Ia.21 | H | Cl | $-CH_2-CH(Cl)-CO-$ | |
| Ia.22 | F | Cl | $-CH_2-CH(Cl)-CO-$ | |
| Ia.23 | H | CN | $-CH_2-CH(Cl)-CO-$ | |
| Ia.24 | F | CN | $-CH_2-CH(Cl)-CO-$ | |

| Nr. | $R^5$ | $R^7$ | X | Schmp. [°C]; IR-Daten ($\gamma$[$cm^{-1}$]); $^1H$-NMR-Daten ($CDCl_3$/TMS, $\delta$ [ppm]) |
|---|---|---|---|---|
| Ia.25 | H | Cl | $-CH=C(Cl)-CO-$ | |
| Ia.26 | F | Cl | $-CH=C(Cl)-CO-$ | |
| Ia.27 | H | CN | $-CH=C(Cl)-CO-$ | |
| Ia.28 | F | CN | $-CH=C(Cl)-CO-$ | |
| Ia.29 | H | Cl | $-CH=C(CH_3)-CO-$ | |
| Ia.30 | F | Cl | $-CH=C(CH_3)-CO-$ | |
| Ia.31 | H | CN | $-CH=C(CH_3)-CO-$ | |
| Ia.32 | F | CN | $-CH=C(CH_3)-CO-$ | |

Des weiteren sind die folgenden substituierten 2-Phenylpyridine der Formel I besonders bevorzugt:

- die Verbindungen Ib.01 - Ib.32, die sich von den entsprechenden Verbindungen Ia.01 - Ia.32 lediglich dadurch unterscheiden, daß Z Tetrahydrofuran-3-yl bedeutet:

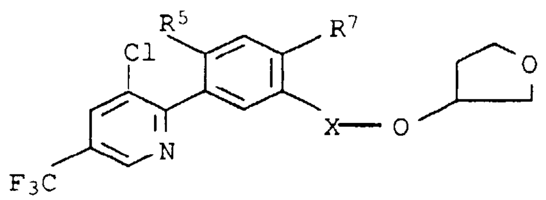

Ib

- die Verbindungen Ic.01 - Ic.32, die sich von den entsprechenden Verbindungen Ia.01 - Ia.32 lediglich dadurch unterscheiden, daß Z eine Gruppe

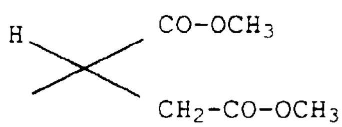

bedeutet:

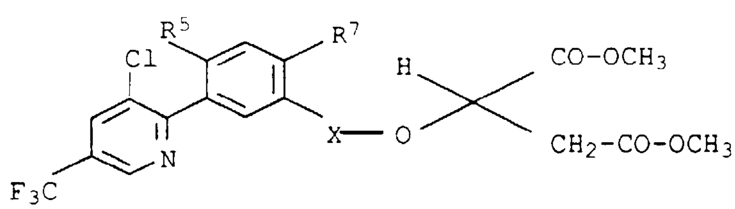

Ic

- die Verbindungen Id.01 - Id.32, die sich von den entsprechenden Verbindungen Ia.01 - Ia.32 lediglich dadurch unterscheiden, daß Z eine Gruppe

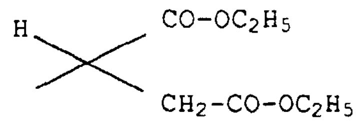

bedeutet:

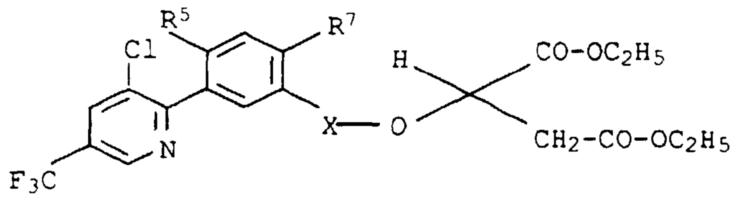
Id

- die Verbindungen Ie.01 - Ie.32, die sich von den entsprechenden Verbindungen Ia.01 - Ia.32 lediglich dadurch unterscheiden, daß Z eine Gruppe

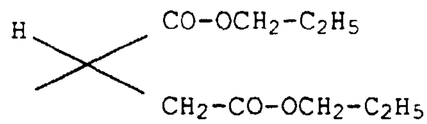

bedeutet:

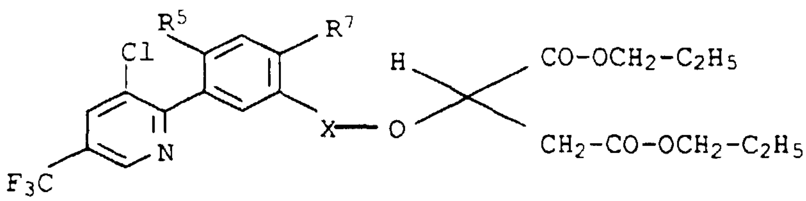
Ie

- die Verbindungen If.01 - If.32, die sich von den entsprechenden Verbindungen Ia.01 - Ia.32 lediglich dadurch unterscheiden, daß Z eine Gruppe

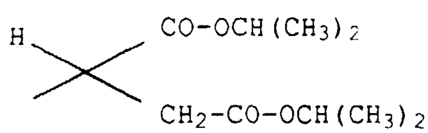

bedeutet:

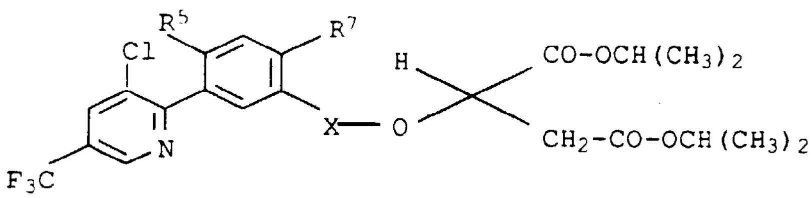
If

Die substituierten 2-Phenylpyridine der Formel I sind auf verschiedene Weise erhältlich, beispielsweise nach einem der folgenden Verfahren:

Verfahren A:

Umsetzung von Säurechloriden III mit 3-Hydroxytetrahydrofuranen IV oder Alkoholen V in Gegenwart einer Base (vgl. z.B. K. Furuta et al., Org. Synth. 72, 86 (1993) und H. Henecka in Houben-Weyl, Methoden der Organischen Chemie, Bd. VIII, 4. Auflage Stuttgart 1952, Seiten 463ff.):

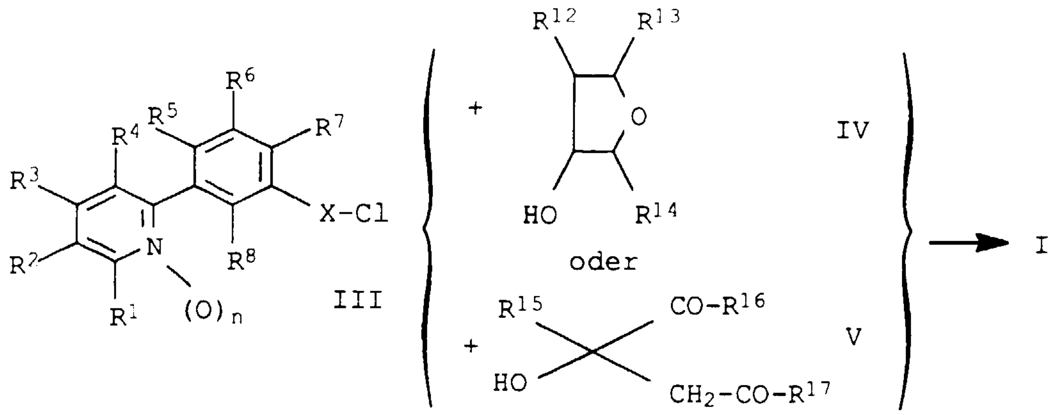


Üblicherweise arbeitet man in einem inerten Lösungs- oder Verdünnungsmittel, insbesondere in einem halogenierten Kohlenwasserstoff wie Dichlormethan, Chloroform, 1,2-Dichlorethan und Tetrachlorkohlenstoff.

Als Basen kommen z.B. Alkalimetall(hydrogen)carbonate wie Natriumhydrogencarbonat und Natriumcarbonat, ferner Stickstoffbasen wie Pyridin, 4-Dimethylaminopyridin und Triethylamin in Betracht.

Die Reaktionstemperatur liegt normalerweise bei 0 bis 100°C.

Üblicherweise werden die Komponenten in etwa stöchiometrischen Mengen eingesetzt, jedoch kann ein Überschuß einer der Komponenten, z.B. im Hinblick auf eine möglichst vollständige Umsetzung der anderen Komponente, vorteilhaft sein.

Die Säurechloride der Formel III sind neu. Zweckmäßigerweise stellt man sie durch Chlorierung der entprechenden freien Carbonsäuren oder deren Alkalimetallsalzen her.

Die Chlorierung kann entweder ohne Lösungsmittel in einem Überschuß des Halogenierungsmittels oder in einem inerten Lösungs- oder Verdünnungsmittel, insbesondere in einem aprotischen Lösungsmittel, z.B. in Diethylether, Benzol oder in Schwefelkohlenstoff, erfolgen.

Als Chlorierungsmittel kommen z.B. Thionylchlorid, Oxalylchlorid, Phosphortrichlorid, Phosphorpentachlorid, Phosphoroxichlorid, Phosgen, Diphosgen oder Triphosgen in Betracht.

Weitere Angaben zur Durchführung derartiger Chlorierungsreaktionen sind in der folgenden Literatur zu finden, auf die beispielhaft verwiesen wird:

- A.J. Meyers und M.E. Flanagan, Org. Synth. 71, 107 (1992);
- H.J. Scheifele Jr. und D.F. DeTar, Org. Synth. Coll. Vol. IV, Seite 34 (1963);
- G.H. Coleman et al., Org. Synth. Coll. Vol. III, Seite 712 (1955);
- H. Henecka in Houben-Weyl, Methoden der Organischen Chemie, Bd. VIII, 4. Auflage Stuttgart 1952, Seiten 463ff.

Die den Säurechloriden III entsprechenden Carbonsäuren sind z.B. aus der DE-A 43 23 916 bekannt oder auf die dort beschriebene Weise erhältlich.

Verfahren B:

Oxidation von substituierten 2-Phenylpyridinen der Formel I, bei denen n Null bedeutet, auf an sich bekannte Weise {vgl. z.B. A. Albini & S. Pietra, Heterocyclic N-Oxides, CRC-Press Inc., Boca Raton, USA 1991: H.S. Mosher et al., Org. Synth. Coll. Vol. IV 1963, Seite 828; E.C. Taylor et al., Org. Synth. Coll. Vol. IV 1963, Seite 704; T.W. Bell et. al., Org. Synth. 69, Seite 226 (1990)}:

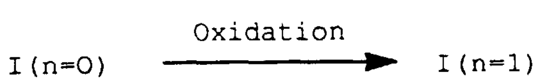

Unter den zur Oxidation des Pyridinrings üblichen Oxidationsmitteln sei beispielhaft auf Peressigsäure, Trifluorperessigsäure, Perbenzoesäure, m-Chlorperbenzoesäure, Monopermaleinsäure, Magnesiummonoperphthalat, Natriumperborat, Oxone® (enthält Peroxidisulfat), Perwolframsäure und Wasserstoffperoxid verwiesen.

Geeignete Lösungsmittel sind z.B. Wasser, Schwefelsäure, Carbonsäuren wie Essigsäure und Trifluoressigsäure sowie halogenierte Kohlenwasserstoffe wie Dichlormethan und Chloroform.

Normalerweise gelingt die Oxidation bei Temperaturen von 0°C bis Siedetemperatur des Reaktionsgemisches.

Das Oxidationsmittel wird normalerweise in mindestens äuqimolaren Mengen, bezogen auf die Ausgangsverbindung, eingesetzt. Im allgemeinen hat sich ein Überschuß an Oxidationsmittel als besonders vorteilhaft erwiesen.

Verfahren C:

Umsetzung von 3-Pyridylphenolen der Formel VII mit Tetrahydrofurylestern VIII oder Estern IX in Gegenwart einer Base:

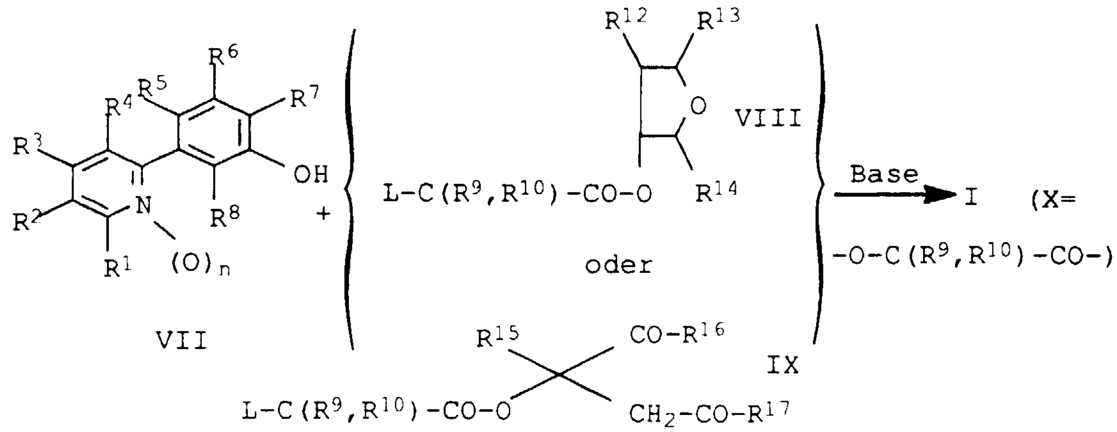


L steht für Chlor, Brom, Jod, Methylsulfonyloxy, Trifluormethylsulfonyloxy, Phenylsulfonyloxy oder p-Tolylsulfonyloxy.

In der Regel arbeitet man in einem inerten Lösungs- oder Verdünnungsmittel, das vorzugsweise aprotisch ist, also z.B. in N,N-Dimethylformamid, Dimethylsulfoxid, Aceton, N-Methylpyrrolidon, Acetonitril oder in einem Ether wie Diethylether, Tetrahydrofuran und 1,4-Dioxan.

Brauchbare Basen sind beispielsweise Alkalimetallcarbonate und -hydrogencarbonate wie Natriumhydrogencarbonat, Kaliumhydrogencarbonat, Natriumcarbonat und Kaliumcarbonat, Alkalimetallalkoholate wie Natriummethanolat und Kalium-tert.-butanolat, Alkalimetallhydroxide wie Natriumhydroxid, und Alkalimetallhydride wie Natriumhydrid.

Weitere Angaben zur Durchführung derartiger Alkylierungsreaktionen sind beispielsweise in der folgenden Literatur zu finden:

* zur Alkylierung von Phenolen mit α-Carbonylsulfonaten:

- U. Burkard und F. Effenberger, Chem. Ber. 119, 1594 (1986);
- J. Bierdermann et al., J. Med. Chem. 29, 1183 (1986);
- R.B. Rogers et al., U.S. 4,725,683.

** zur Alkylierung von Phenolen mit α-Halogenestern:

- R. Aneja et al., Tetrahedron 2, 203 (1958);
- EP-A 380 043;
- C.R. Edwards et al., J. Heterocycl. Chem. 24, 495 (1987);
- C.P. Phadke et al., Synthesis 5, 413 (1986);
- K.G. Watson, U.S. 4,837,355;
- V. Elango et al., U.S. 4,908,476;
- G. Schlegel et al., U.S. 4,978,774;
- U. Burkard und F. Effenberger, Chem. Ber. 119, 1594 (1986);
- H. Sugihara et al., Chem. And Pharm. Bull. 35, 1919 (1987);

- S. Fujinawa et al., U.S. 4,625,053.

Die Tetrahydrofurylester VIII und Ester IX sind bekannt oder auf an sich bekannte Weise erhältlich {siehe z.B. EP-A 548 593, U.S. 4,086,076 sowie H.G. Zachau und W. Krau, Chem. Ber. 93, 1830 (1960)}.

Verfahren D:

Umsetzung von 3-Pyridylthiophenolen der Formel X mit Tetrahydrofurylestern VIII oder Estern IX in Gegenwart einer Base:

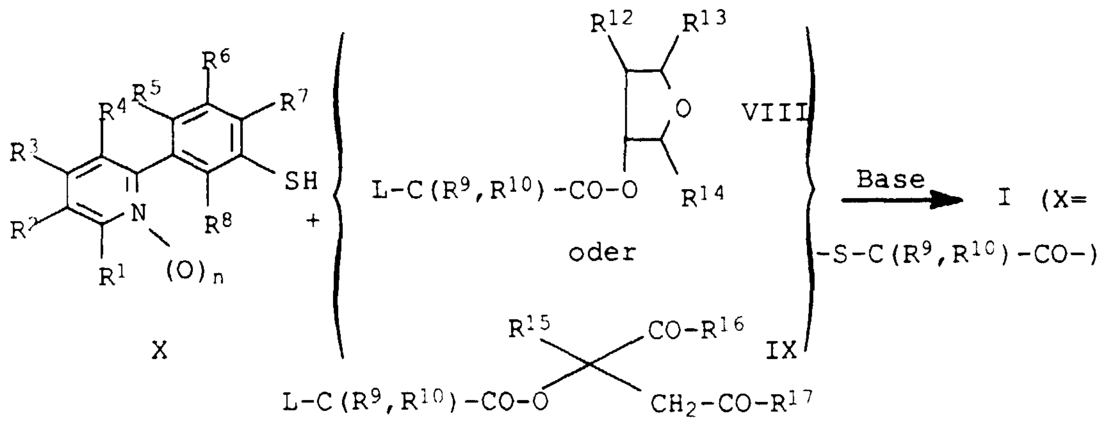

X

VIII

oder

IX

Bezüglich der Definition von L sowie geeigneter Lösungs-/Verdünnungsmittel und Basen gelten die Angaben für Verfahren C.

Weitere Angaben zur Durchführung derartiger Alkylierungsreaktionen sind beispielsweise in der folgenden Literatur zu finden:

* zur Alkylierung von Thiophenolen mit α-Carbonylsulfonaten:

- U. Burkard und F. Effenberger, Chem. Ber. 119, 1594 (1986);

** zur Alkylierung von Thiophenolen mit α-Halogenestern:

- M.B. Floyd, U.S. 4,983,753;
- E. Campaigne und A.R. McLaughlin, J. Heterocycl. Chem. 20, 623 (1983);
- J. Durman et al., J. Chem. Soc. Perkin Trans., 1939 (1986);
- M. Kawada et al., Chem. Pharm. Bull. 34, 1939 (1986);
- H. Sugihara et al., Chem. And Pharm. Bull. 35, 1919 (1987).

Sofern nicht anders angegeben werden alle vorstehend beschriebenen Verfahren zweckmäßigerweise bei Atmosphärendruck oder unter dem Eigendruck des jeweiligen Reaktionsgemisches vorgenommen.

Die Aufarbeitung der Reaktionsgemische erfolgt in der Regel nach an sich bekannten Methoden, beispielsweise durch Entfernen des Lösungsmittels, Verteilen des Rückstandes in einem Gemisch aus Wasser und einem geeigneten organischen Lösungsmittel und Aufarbeiten der organischen Phase auf das Produkt hin.

Die substituierten 2-Phenylpyridine der Formel I können ein oder mehrere Chiralitätszentren enthalten und fallen dann üblicherweise als Enantiomeren- oder Diastereomerengemische an. Die Mischungen können gewünschtenfalls nach den hierfür üblichen Methoden, z.B. mittels Kristallisation oder Chromatographie an einem optisch aktiven Adsorbat, in die weitgehend reinen Isomeren getrennt werden. Reine optisch aktive Isomere lassen sich beispielsweise auch aus entsprechenden optisch aktiven Ausgangsmaterialien herstellen.

Substituierte 2-Phenylpyridine I mit CH-aciden Substituenten lassen sich auf an sich bekannte Weise in ihre Salze, vorzugsweise in ihre Alkalimetallsalze, überführen.

Salze von I, deren Metallion kein Alkalimetallion ist, können durch Umsalzen des entsprechenden Alkalimetallsalzes in üblicher Weise hergestellt werden, ebenso Ammonium- und Phosphoniumsalze mittels Ammoniak, Phosphonium-, Sulfonium- oder Sulfoxoniumhydroxiden.

Diejenigen Verbindungen I, die eine endständige Aminogruppe tragen, können ferner Säureadditionssalze bilden.

Die Verbindungen I und deren landwirtschaftlich brauchbaren Salze eignen sich - sowohl als Isomerengemische als auch in Form der reinen Isomeren - als Herbizide. Die I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen: Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassiza rapa var. silvestris, Camellia sinensis, Carthamus tinctorius, Carya illinoinensis, Citrus limon, Citrus sinensis, coffea arabica (Coffea canephora, Coffea liberica), Cucumis Sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa , Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum, tuberosum, Sorghum bicolor (s. vulgare), Theobroma cacao, Trifolium pratense, Triticum aestivum, Triticum durum, Vicia faba, Vitis vinifera und Zea mays.

Darüber hinaus können die Verbindungen I auch in Kulturen, die durch Züchtung einschließlich gentechnischer Methoden gegen die Wirkung von Herbiziden tolerant sind, verwandt werden.

Des weiteren eignen sich die substituierten 2-Phenylpyridine I auch zur Desikkation und/oder Defoliation von Pflanzen.

Als Desikkantien eignen sie sich insbesondere zur Austrocknung der oberirdischen Teile von Kulturpflanzen wie Kartoffel, Raps, Sonnenblume und Sojabohne. Damit wird ein vollständig mechanisches Beernten dieser wichtigen Kulturpflanzen ermöglicht.

Von wirtschaftlichem Interesse ist ferner die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- oder Blatt- und Sproßteil der Pflanzen ist auch für ein gut kontrollierbares Entblättern von Nutzpflanzen, insbesondere Baumwolle, wesentlich.

Außerdem führt die Verkürzung des Zeitintervalls, in dem die einzelnen Baumwollpflanzen reif werden, zu einer erhöhten Faserqualität nach der Ernte.

Die Verbindungen I bzw. die sie enthaltenden Mittel können beispielsweise in Form von direkt versprühbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Hilfsstoffe für die Herstellung von direkt versprühbaren Lösungen, Emulsionen, Pasten oder Öldispersionen kommen im wesentlichen in Betracht: Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe (Adjuvantien) kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Im allgemeinen enthalten die Formulierungen von 0,001 bis 98 Gew.%, vorzugsweise 0,01 bis 95 Gew.%, Wirkstoff. Die Wirkstoffe werden dabei in einer Reinheit von 90% bis 100%, vorzugsweise 95% bis 100% (nach NMR-Spektrum) eingesetzt.

Die folgenden Formulierungsbeispiele verdeutlichen die Herstellung solcher Zubereitungen:

I. 20 Gewichtsteile der Verbindung Nr. Ia.10 werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

II. 20 Gewichtsteile der Verbindung Nr. Ia.10 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

III. 20 Gewichtsteile des Wirkstoffs Nr. Ia.10 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. Ia.10 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser enthält man eine Spritzbrühe, die 0,1 Gew.% des Wirkstoffs enthält.

V. 3 Gewichtsteile des Wirkstoffs Nr. Ia.10 werden mit 97 Gewichtsteilen feinteiligem Kaolin vermischt. Man erhält auf diese Weise ein Stäubemittel, das 3 Gew.% des Wirkstoffs enthält.

VI. 20 Gewichtsteile des Wirkstoffs Nr. Ia.10 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondensates und 68 Gewichtsteilen eines paraffinischen Mineralöls innig vermischt. Man erhält eine stabile ölige Dispersion.

VII. 1 Gewichtsteil der Verbindung Nr. Ia.10 wird in einer Mischung gelöst, die aus 70 Gewichtsteilen Cyclohexanon, 20 Gewichtsteilen ethoxyliertem Isooctylphenol und 10 Gewichtsteilen ethoxyliertem Ricinusöl besteht. Man erhält ein stabiles Emulsionskonzentrat.

VIII. 1 Gewichtsteil der Verbindung Nr. Ia.10 wird in einer Mischung gelöst, die aus 80 Gewichtsteilen Cyclohexanon und 20 Gewichtsteilen Emulphor EL[1] besteht. Man erhält ein stabiles Emulsionskonzentrat.

Die Applikation der Wirkstoffe I bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der

[1] ethoxyliertes Ricinusöl (caster-oil)

empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff I betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

Zur Verbreiterung des Wirkungsspektrums und zur Erzielung synergistischer Effekte können die substituierten 2-Phenylpyridine I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF3-Phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile in Betracht.

Außerdem kann es von Nutzen sein, die Verbindungen I allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Herstellungsbeispiel

Verbindung Nr. Ia.10 in Tabelle 1

Die Herstellung erfolgte gemäß folgender Reaktionsgleichung:

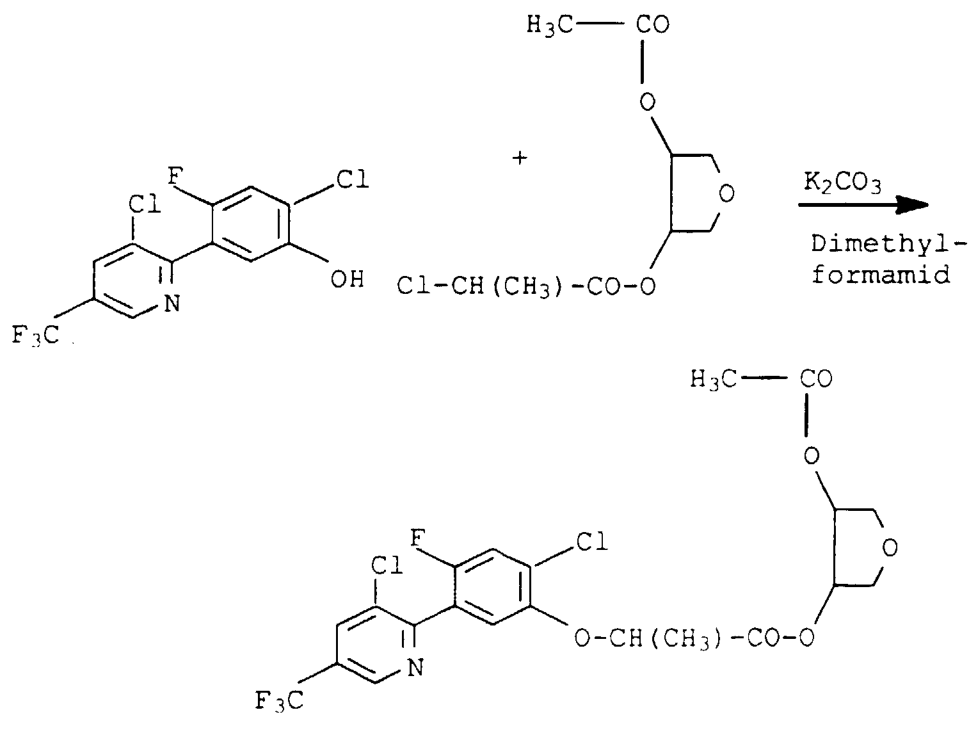


1,0 g 3-Chlor-2-(4-chlor-2-fluor-5-hydroxyphenyl)-5-trifluormethylpyridin, 1,1 g (±)-(3R,4S)-4-Acetoxy-3-(2-chlorpropionyloxy)-tetrahydrofuran und 0,85 g Kaliumcarbonat wurden in 50 ml wasserfreiem Dimethylformamid drei Stunden bei 80°C gerührt. Dann wurde der erkaltete Reaktionsansatz auf 200 ml Eiswasser gegossen, wonach man dreimal mit je 100 ml tert.-Butylmethylether extrahierte. Die vereinigten organischen Phasen wurden zweimal mit je 50 ml Wasser gewaschen, über Natriumsulfat getrocknet und eingeengt. Ausbeute: 1,3 g (80%) eines farblosen Öls (Dia-

stereomerengemisch).
$^1H$-NMR (270 MHz, in $CDCl_3$): δ[ppm] =1.67(d,3H; Nebendiastereomer), 1.71(d,3H; Hauptdiastereomer), 1.97(s,3H; Nebendiastereomer), 2.00(s,3H; Hauptdiastereomer), 3.70-4.11(m,4H), 4.82(q,1H), 5.30(q,1H), 5.36-5,44(m,1H), 7.06 (d,1H), 7.29(d,1H), 8.05(s,1H), 8.84(s,1H).

Vorstufe:

(±)- (3R,4S)-4-Acetoxy-3-(2-chlorpropionyloxy)-tetrahydrofuran

Zu einer mittels Eis gekühlten Mischung aus 15,0 g (±)-(3R,4S)--3-Hydroxy-4-methylcarbonyloxy-tetrahydrofuran, 8,1 g wasserfreiem Pyridin und 200 ml wasserfreiem Dichlormethan wurden bei 0-5°C 12,4 g (±)-2-Chlorpropionsäurechlorid getropft. Nach beendeter Zugabe rührte man noch 4 Stunden bei 23°C, wonach das Reaktionsgemisch auf 400 ml Wasser gegossen wurde. Anschließend wurde die organische Phase abgetrennt, zweimal mit je 50 ml Wasser gewaschen, über Natriumsulfat getrocknet und schließlich eingeengt. Das Rohprodukt reinigte man destillativ bei 10 mbar Druck. Ausbeute: 15,7 g (68 %) eines farblosen Öls.
$^1H$-NMR (270 MHz, in $CDCl_3$): δ[ppm] = 1.73(d,3H), 2.09(s,3H), 3.80-3.92(m,2H), 4.07-4.16(m,2H), 4.45(q,1H), 5.33-5.44(m,2H).

Anwendungsbeispiele (herbizide Wirksamkeit)

Die herbizide Wirkung der substituierten 2-Phenylpyridine I ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 0,0078 oder 0,0039 kg/ha a.S. (aktive Substanz).

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Lateinischer Name | Deutscher Name | Englischer Name |
|---|---|---|
| Abutilon theophrasti | Chinesischer Hanf | velvet leaf |
| Amaranthus retroflexus | Zurückgekrümmter Fuchsschwanz | redroot pigweed |
| Ipomoea subspecies | Prunkwindearten | morningglory |
| Solanum nigrum | Schwarzer Nachtschatten | black nightshade |

Bei einer Aufwandmenge von 0,0078 oder 0,0039 kg/ha a.S. zeigte die Verbindung Nr. Ia.10 im Nachauflaufverfahren eine sehr gute Wirkung gegen die o.g. Pflanzen.

Anwendungsbeispiele (desikkative/defoliante Wirksamkeit)

Als Testpflanzen dienten junge, 4-blättrige (ohne Keimblätter) Baumwollpflanzen, die unter Gewächshausbedingungen angezogen wurden (rel. Luftfeuchtigkeit 50 bis 70 %; Tag-/Nachttemperatur 27/20°C).

Die jungen Baumwollpflanzen wurden tropfnaß mit wässrigen Aufbereitungen der Wirkstoffe (unter Zusatz von

0,15 Gew.-% des Fettalkoholalkoxylats Plurafac LF 700, bezogen auf die Spritzbrühe) blattbehandelt. Die ausgebrachte Wassermenge betrug umgerechnet 1000 1/ha. Nach 13 Tagen wurde die Anzahl der abgeworfenen Blätter und der Grad der Entblätterung in % bestimmt.

Bei den unbehandelten Kontrollpflanzen trat kein Blattfall auf.

**Patentansprüche**

1. Substituierte 2-Phenylpyridine der allgemeinen Formel I

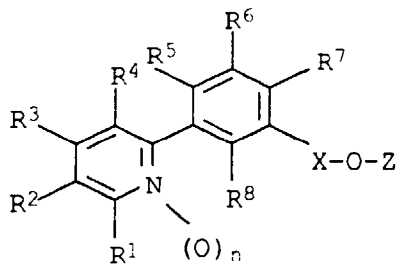

I

in der die Variablen folgende Bedeutungen haben:

| | |
|---|---|
| n | 0 oder 1; |
| $R^1$, $R^3$, $R^4$ | unabhängig voneinander Wasserstoff, Halogen, $C_1$-$C_4$-Alkyl, $C_1$-$C_4$-Halogenalkyl, Hydroxy, $C_1$-$C_4$-Alkoxy, $C_1$-$C_4$-Halogenalkoxy, Nitro, Amino, $C_1$-$C_4$-Alkylamino, Di-($C_1$-$C_4$-alkyl)-amino, Mercapto, $C_1$-$C_4$-Alkylthio, C1-C4-Halogenalkylthio, Cyano, Carboxyl, ($C_1$-$C_4$-Alkoxy)carbonyl, Aminocarbonyl, $C_1$-$C_4$-Alkylamino-carbonyl oder Di-($C_1$-$C_4$-alkyl)-aminocarbonyl; |
| $R^2$ | Wasserstoff, Halogen, $C_1$-$C_4$-Alkyl, $C_1$-$C_4$-Halogenalkyl, Cyano, Nitro, Amino, Hydroxy, $C_1$-$C_4$-Alkoxy, $C_1$-$C_4$-Halogen-alkoxy, Mercapto, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl; |
| $R^5$ | Wasserstoff, Halogen oder Cyano; |
| $R^6$ und $R^8$ | unabhängig voneinander Wasserstoff oder Halogen; |
| $R^7$ | Wasserstoff, Cyano, Nitro, Hydroxy, Trifluormethylsulfonyloxy, Halogen, $C_1$-$C_4$-Alkyl, $C_1$-$C_4$-Halogenalkyl, $C_1$-$C_4$-Alkoxy oder $C_1$-$C_4$-Halogenalkoxy; |
| X | eine der folgenden Brücken: -CO-, -O-C($R^9$,$R^{10}$)-CO-, -S-C($R^9$,$R^{10}$)-CO-, -$CH_2$-CH($R^{11}$)-CO- oder -CH=C($R^{11}$)-CO-, wobei $R^9$ und $R^{10}$ unabhängig voneinander für Wasserstoff oder $C_1$-$C_4$-Alkyl und $R^{11}$ für Wasserstoff, Halogen oder $C_1$-$C_4$-Alkyl stehen; |
| Z | eine Gruppe |

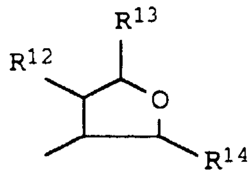

oder

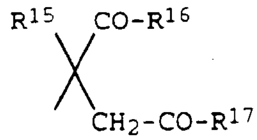

,

wobei

$R^{12}$ für Wasserstoff, Hydroxy, Halogen, $C_1$-$C_4$-Alkyl, $C_1$-$C_4$-Alkoxy, ($C_1$-$C_4$-Alkyl)carbonyloxy, ($C_1$-$C_4$-Halogenalkyl)carbonyloxy, ($C_3$-$C_6$-Cycloalkyl)carbonyloxy, Benzoyloxy, Benzylcarbonyloxy, Benzyloxy, ($C_1$-$C_4$-Alkoxy)carbonyloxy, ($C_1$-$C_4$-Alkylamino)carbonyloxy, Di-($C_1$-$C_4$-alkyl)-aminocarbonyloxy oder $C_1$-$C_3$-Alkylsulfonyloxy;

$R^{13}$ und $R^{14}$ unabhängig voneinander für Wasserstoff, Hydroxy, $C_1$-$C_4$-Alkyl, $C_1$-$C_4$-Alkoxy oder ($C_1$-$C_4$-Alkyl)carbonyloxy;

$R^{15}$ für Wasserstoff oder $C_1$-$C_4$-Alkyl und

$R^{16}$ und $R^{17}$ unabhängig voneinander für Hydroxy, $C_1$-$C_6$-Alkoxy, $C_3$-$C_6$-Alkenyloxy, $C_3$-$C_6$-Alkinyloxy, $C_1$-$C_6$-Alkoxy-$C_2$-$C_4$-alkoxy, $C_3$-$C_6$-Cycloalkyloxy, Amino, $C_1$-$C_4$-Alkylamino, Di-($C_1$-$C_4$-alkyl)-amino, N-Pyrrolidinyl, N-Piperidinyl, N-Morpholinyl, $C_3$-$C_6$-Cycloalkylamino, Di-($C_3$-$C_6$-cycloalkyl)-amino, $C_3$-$C_6$-Alkenylamino oder $C_3$-$C_6$-Alkinylamino oder

$R^{16}$ und $R^{17}$ zusammen für eine Brücke -N($R^{18}$)-N($R^{19}$)- oder -N($R^{20}$)- stehen,

wobei $R^{18}$ und $R^{19}$ jeweils Wasserstoff oder $C_1$-$C_4$-Alkyl und

$R^{20}$ Wasserstoff, $C_1$-$C_6$-Alkyl, $C_3$-$C_6$-Alkenyl, $C_3$-$C_6$-Alkinyl, $C_1$-$C_6$-Alkoxy oder $C_3$-$C_6$-Alkenyloxy

bedeuten,
sowie die landwirtschaftlich brauchbaren Salze der Verbindungen I, sofern diese existieren.

**2.** Substituierte 2-Phenylpyridine der Formel I nach Anspruch 1, wobei n für null, $R^1$, $R^3$ und $R^4$ unabhängig voneinander für Wasserstoff oder Halogen, $R^2$ für Halogen oder $C_1$-$C_4$-Halogenalkyl mit ein bis fünf Halogenatomen, $R^5$ für Wasserstoff oder Halogen, $R^6$ für Wasserstoff, $R^7$ für Halogen oder Cyano, $R^8$ für Wasserstoff, $R^9$ und $R^{10}$ unabhängig voneinander für Wasserstoff oder $C_1$-$C_4$-Alkyl, $R^{11}$ für Wasserstoff oder Halogen, $R^{12}$, $R^{13}$, $R^{14}$ unabhängig voneinander für Wasserstoff, $C_1$-$C_4$-Alkyl, Hydroxy oder ($C_1$-$C_4$-Alkyl)carbonyloxy, $R^{15}$ für Wasserstoff, $R^{16}$ und $R^{17}$ unabhängig voneinander für $C_1$-$C_6$-Alkoxy, $C_3$-$C_6$-Alkenyloxy, Amino, $C_1$-$C_4$-Alkylamino, Di-($C_1$-$C_4$-alkyl)-amino oder zusammen für eine Brücke -NH-NH-, -N($C_1$-$C_4$-Alkyl)-N($C_1$-$C_4$-Alkyl)- oder -N($R^{20}$)- stehen und $R^{20}$ Wasserstoff oder $C_1$-$C_6$-Alkyl bedeutet.

**3.** Verwendung der substituierten 2-Phenylpyridine der Formel I und der landwirtschaftlich brauchbaren Salze von I, gemäß Anspruch 1, als Herbizide oder zur Desikkation und/oder Defoliation von Pflanzen.

**4.** Herbizides Mittel, enthaltend eine herbizid wirksame Menge mindestens eines substituierten 2-Phenylpyridins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

**5.** Mittel zur Desikkation und/oder Defoliation von Pflanzen, enthaltend eine desikkant und/oder defoliant wirksame Menge mindestens eines substituierten 2-Phenylpyridins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff.

**6.** Verfahren zur Herstellung von herbizid wirksamen Mitteln, dadurch gekennzeichnet, daß man eine herbizid wirksame Menge mindestens eines substituierten 2-Phenylpyridins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

**7.** Verfahren zur Herstellung von desikkant und/oder defoliant wirksamen Mitteln, dadurch gekennzeichnet, daß man eine desikkant und/oder defoliant wirksame Menge mindestens eines substituierten 2-Phenylpyridins der Formel I oder eines landwirtschaftlich brauchbaren Salzes von I, gemäß Anspruch 1, und mindestens einen inerten flüssigen und/oder festen Trägerstoff sowie gewünschtenfalls mindestens einen oberflächenaktiven Stoff mischt.

**8.** Verfahren zur Bekämpfung von unerwünschtem Pflanzenwuchs, dadurch gekennzeichnet, daß man eine herbizid wirksame Menge mindestens eines substituierten 2-Phenylpyridins der Formel I oder eines landwirtschaftlich

brauchbaren Salzes von I, gemäß Anspruch 1, auf Pflanzen, deren Lebensraum oder auf Saatgut einwirken läßt.

**9.** Verfahren zur Desikkation und/oder Defoliation von Pflanzen, dadurch gekennzeichnet, daß man eine desikkant und/oder defoliant wirksame Menge mindestens eines substituierten 2-Phenylpyridins der Formel I oder eines landwirtschaftlich brauchbaren Salzes von I, gemäß Anspruch 1, auf Pflanzen einwirken läßt.

**10.** Verfahren nach Anspruch 9, dadurch gekennzeichnet, daß man Baumwolle behandelt.

**11.** Verfahren zur Herstellung von substituierten 2-Phenylpyridinen der Formel I gemäß Anspruch 1, bei denen Z die Gruppe

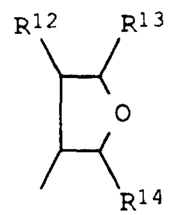

bedeutet, dadurch gekennzeichnet, daß man ein Säurechlorid der Formel III

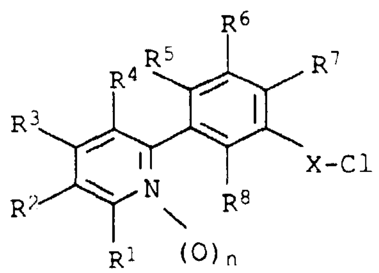

III

auf an sich bekannte Weise in einem inerten Lösungs- oder Verdünnungsmittel, in Gegenwart einer Base, mit einem 3-Hydroxytetrahydrofuran der Formel IV

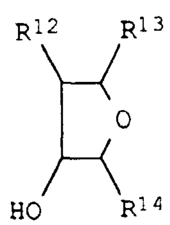

IV

umsetzt.

**12.** Verfahren zur Herstellung von substituierten 2-Phenylpyridinen der Formel I gemäß Anspruch 1, bei denen Z die Gruppe

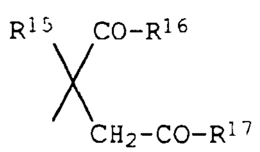

bedeutet, dadurch gekennzeichnet, daß man ein Säurechlorid der Formel III

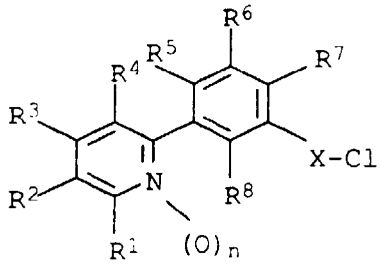

III

auf an sich bekannte Weise in einem inerten Lösungs- oder Verdünnungsmittel, in Gegenwart einer Base, mit einem Alkohol der Formel V

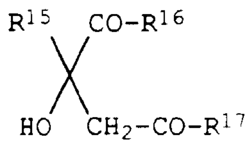

V

umsetzt.

**13.** Säurechloride der Formel III

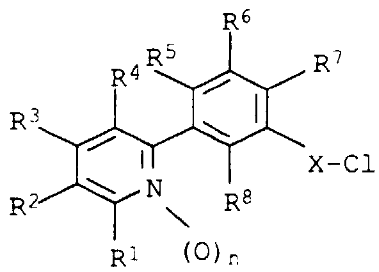

III

in der die Variablen folgende Bedeutung haben:

| | |
|---|---|
| n | 0 oder 1; |
| $R^1$, $R^3$, $R^4$ | unabhängig voneinander Wasserstoff, Halogen, $C_1$-$C_4$-Alkyl, $C_1$-$C_4$-Halogenalkyl, Hydroxy, $C_1$-$C_4$-Alkoxy, $C_1$-$C_4$-Halogenalkoxy, Nitro, Amino, $C_1$-$C_4$-Alkylamino, Di-($C_1$-$C_4$-alkyl)-amino, Mercapto, $C_1$-$C_4$-Alkylthio, $C_1$-$C_4$-Halogenalkylthio, Cyano, Carboxyl, ($C_1$-$C_4$-Alkoxy)carbonyl, Aminocarbonyl, $C_1$-$C_4$-Alkylaminocarbonyl oder Di-($C_1$-$C_4$-alkyl)-aminocarbonyl; |
| $R^2$ | Wasserstoff, Halogen, $C_1$-$C_4$-Alkyl, $C_1$-$C_4$-Halogenalkyl, Cyano, Nitro, Amino, Hydroxy, $C_1$-$C_4$-Alkoxy, $C_1$-$C_4$-Halogenalkoxy, Mercapto, $C_1$-$C_4$-Alkylthio, $C_1$-$C_4$-Halogenalkylthio, $C_1$-$C_4$-Alkylsulfinyl, $C_1$-$C_4$-Halogenalkylsulfinyl, $C_1$-$C_4$-Alkylsulfonyl oder $C_1$-$C_4$-Halogenalkylsulfonyl; |
| $R^5$ | Wasserstoff, Halogen oder Cyano; |
| $R^6$ und $R^8$ | unabhängig voneinander Wasserstoff oder Halogen; |
| $R^7$ | Wasserstoff, Cyano, Nitro, Hydroxy, Trifluormethylsulfonyloxy, Halogen, $C_1$-$C_4$-Alkyl, $C_1$-$C_4$-Halogenalkyl, $C_1$-$C_4$-Alkoxy oder $C_1$-$C_4$-Halogenalkoxy; |

X eine der folgenden Brücken:
-CO-, -O-C($R^9$,$R^{10}$)-CO-, -S-C($R^9$,$R^{10}$)-CO-, -$CH_2$-CH($R^{11}$)-CO- oder -CH=C($R^{11}$)-CO-, wobei $R^9$ und $R^{10}$ unabhängig voneinander für Wasserstoff oder $C_1$-$C_4$-Alkyl und $R^{11}$ für Wasserstoff, Halogen oder $C_1$-$C_4$-Alkyl stehen.

**14.** Verfahren zur Herstellung der Säurechloride der Formel III gemäß Anspruch 13, dadurch gekennzeichnet, daß man die entsprechenden freien Carbonsäuren oder deren Alkalimetallsalze auf an sich bekannte Weise chloriert.

**15.** Verfahren zur Herstellung von substituierten 2-Phenylpyridinen der Formel I gemäß Anspruch 1, wobei n für 1 steht, dadurch gekennzeichnet, daß man die entsprechenden substituierten 2-Phenylpyridine, bei denen n null bedeutet, auf an sich bekannte Weise in einem inerten Lösungs- oder Verdünnungsmittel oxidiert.

**16.** Verfahren zur Herstellung von substituierten 2-Phenylpyridinen der Formel I gemäß Anspruch 1, wobei X eine Brücke -O-C($R^9$,$R^{10}$)-CO- bedeutet, dadurch gekennzeichnet, daß man ein 3-Pyridylphenol der Formel VI

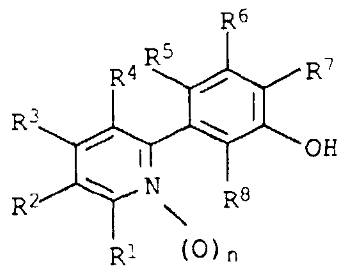

VI

auf an sich bekannte Weise in einem inerten Lösungs- oder Verdünnungsmittel, in Gegenwart einer Base, mit einem Tetrahydrofurylester der Formel VII

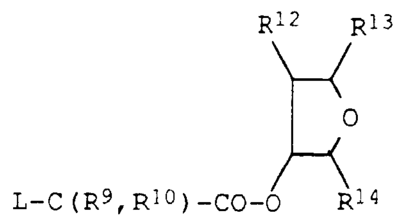

VII

oder einem Ester der Formel VIII

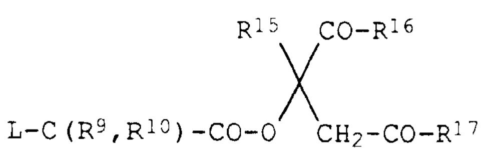

VIII

wobei L jeweils für Chlor, Brom, Jod, Methylsulfonyloxy, Trifluormethylsulfonyloxy, Phenylsulfonyloxy oder p-Toluolsulfonyloxy steht, umsetzt.

**17.** Verfahren zur Herstellung von substituierten 2-Phenylpyridinen der Formel I gemäß Anspruch 1, wobei X eine Brücke -S-C($R^9$,$R^{10}$)-CO- bedeutet, dadurch gekennzeichnet, daß man ein 3-Pyridylthiophenol der Formel IX

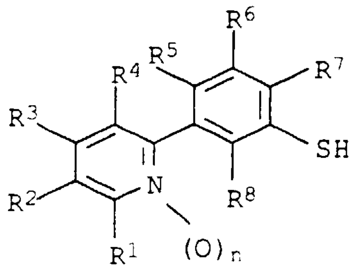

IX

auf an sich bekannte Weise in einem inerten Lösungs- oder Verdünnungsmittel, in Gegenwart einer Base, mit einem Tetrahydrofurylester der Formel VII

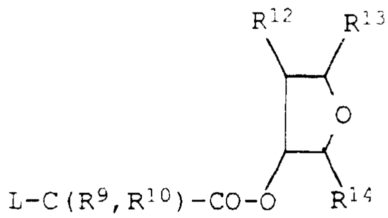

VII

oder einem Ester der Formel VIII

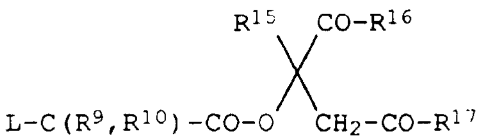

VIII

wobei L jeweils für Chlor, Brom, Jod, Methylsulfonyloxy, Trifluormethylsulfonyloxy, Phenylsulfonyloxy oder p-Tolylsulfonyloxy steht, umsetzt.

## Claims

**1.** A substituted 2-phenylpyridine of the general formula I

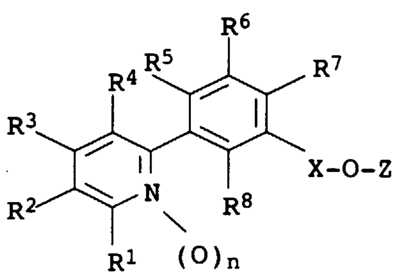

I

where the variables have the following meanings:

n — is 0 or 1;

$R^1$, $R^3$ and $R^4$ — independently of one another are hydrogen, halogen, $C_1$-$C_4$-alkyl, $C_1$-$C_4$-haloalkyl, hydroxyl, $C_1$-$C_4$-alkoxy, $C_1$-$C_4$-haloalkoxy, nitro, amino, $C_1$-$C_4$-alkylamino, di-($C_1$-$C_4$-alkyl)-amino, mercapto, $C_1$-$C_4$-alkylthio, $C_1$-$C_4$-haloalkylthio, cyano, carboxyl, ($C_1$-$C_4$-alkoxy)carbonyl, aminoc-

arbonyl, $C_1$-$C_4$-alkylaminocarbonyl or di-($C_1$-$C_4$-alkyl)-aminocarbonyl;

$R^2$ is hydrogen, halogen, $C_1$-$C_4$-alkyl, $C_1$-$C_4$-haloalkyl, cyano, nitro, amino, hydroxyl, $C_1$-$C_4$-alkoxy, $C_1$-$C_4$-haloalkoxy, mercapto, $C_1$-$C_4$-alkylthio, $C_1$-$C_4$-haloalkylthio, $C_1$-$C_4$-alkylsulfinyl, $C_1$-$C_4$-haloalkylsulfinyl, $C_1$-$C_4$-alkylsulfonyl or $C_1$-$C_4$-haloalkylsulfonyl;

$R^5$ is hydrogen, halogen or cyano;

$R^6$ and $R^8$ independently of one another are hydrogen or halogen;

$R^7$ is hydrogen, cyano, nitro, hydroxyl, trifluoromethylsulfonyloxy, halogen, $C_1$-$C_4$-alkyl, $C_1$-$C_4$-haloalkyl, $C_1$-$C_4$-alkoxy or $C_1$-$C_4$-haloalkoxy;

X is one of the following bridges:
-CO-, -O-C($R^9$,$R^{10}$)-CO-, -S-C($R^9$,$R^{10}$)-CO-, -$CH_2$-CH($R^{11}$)-CO- or -CH=C($R^{11}$)-CO-, where $R^9$ and $R^{10}$ independently of one another are hydrogen or $C_1$-$C_4$-alkyl and $R^{11}$ is hydrogen, halogen or $C_1$-$C_4$-alkyl;

Z is a group

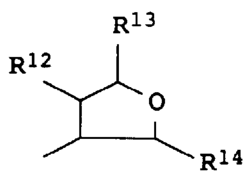

or

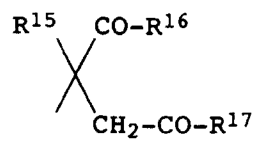

,

where

$R^{12}$ is hydrogen, hydroxyl, halogen, $C_1$-$C_4$-alkyl, $C_1$-$C_4$-alkoxy, ($C_1$-$C_4$-alkyl)carbonyloxy, ($C_1$-$C_4$-haloalkyl)carbonyloxy, ($C_3$-$C_6$-cycloalkyl)carbonyloxy, benzoyloxy, benzylcarbonyloxy, benzyloxy, ($C_1$-$C_4$-alkoxy)carbonyloxy, ($C_1$-$C_4$-alkylamino)carbonyloxy, di-($C_1$-$C_4$-alkyl)-aminocarbonyloxy or $C_1$-$C_3$-alkylsulfonyloxy;

$R^{13}$ and $R^{14}$ independently of one another are hydrogen, hydroxyl, $C_1$-$C_4$-alkyl, $C_1$-$C_4$-alkoxy or ($C_1$-$C_4$-alkyl)carbonyloxy;

$R^{15}$ is hydrogen or $C_1$-$C_4$-alkyl and

$R^{16}$ and $R^{17}$ independently of one another are hydroxyl, $C_1$-$C_6$-alkoxy, $C_3$-$C_6$-alkenyloxy, $C_3$-$C_6$-alkynyloxy, $C_1$-$C_6$-alkoxy-$C_2$-$C_4$-alkoxy, $C_3$-$C_6$-cycloalkyloxy, amino, $C_1$-$C_4$-alkylamino, di-($C_1$-$C_4$-alkyl)-amino, N-pyrrolidinyl, N-piperidinyl, N-morpholinyl, $C_3$-$C_6$-cycloalkylamino, di-($C_3$-$C_6$-cycloalkyl)-amino, $C_3$-$C_6$-alkenylamino or $C_3$-$C_6$-alkynylamino or

$R^{16}$ and $R^{17}$ together are a bridge -N($R^{18}$)-N($R^{19}$)- or -N($R^{20}$)-,

where $R^{18}$ and $R^{19}$ are in each case hydrogen or $C_1$-$C_4$-alkyl and

$R^{20}$ is hydrogen, $C_1$-$C_6$-alkyl, $C_3$-$C_6$-alkenyl, $C_3$-$C_6$-alkynyl, $C_1$-$C_6$-alkoxy or $C_3$-$C_6$-alkenyloxy,

or the agriculturally utilizable salts of the compounds I, if these exist.

**2.** A substituted 2-phenylpyridine of the formula I as claimed in claim 1, where n is zero, $R^1$, $R^3$ and $R^4$ independently of one another are hydrogen or halogen, $R^2$ is halogen or $C_1$-$C_4$-haloalkyl having one to five halogen atoms, $R^5$ is hydrogen or halogen, $R^6$ is hydrogen, $R^7$ is halogen or cyano, $R^8$ is hydrogen, $R^9$ and $R^{10}$ independently of one another are hydrogen or $C_1$-$C_4$-alkyl, $R^{11}$ is hydrogen or halogen, $R^{12}$, $R^{13}$ and $R^{14}$ independently of one another are hydrogen, $C_1$-$C_4$-alkyl, hydroxyl or ($C_1$-$C_4$-alkyl)carbonyloxy, $R^{15}$ is hydrogen, $R^{16}$ and $R^{17}$ independently of

one another are $C_1$-$C_6$-alkoxy, $C_3$-$C_6$-alkenyloxy, amino, $C_1$-$C_4$-alkylamino, di-($C_1$-$C_4$-alkyl)amino or together are a bridge -NH-NH-, -N($C_1$-$C_4$-Alkyl)-N($C_1$-$C_4$-alkyl)- or -N($R^{20}$)- and $R^{20}$ is hydrogen or $C_1$-$C_6$-alkyl.

**3.** The use of the substituted 2-phenylpyridines of the formula I and of the agriculturally utilizable salts of I, as claimed in claim 1, as herbicides or for the dessication and/or defoliation of plants.

**4.** A herbicidal composition containing a herbicidally active amount of at least one substituted 2-phenylpyridine of the formula I or of an agriculturally utilizable salt of I, as claimed in claim 1, and at least one inert liquid and/or solid carrier and also, if desired, at least one surface-active substance.

**5.** A composition for the desiccation and/or defoliation of plants, containing an amount of at least one substituted 2-phenylpyridine of the formula I or of an agriculturally utilizable salt of I, as claimed in claim 1, which has desiccant and/or defoliant activity, and at least one inert liquid and/or solid carrier and also, if desired, at least one surface-active substance.

**6.** A process for preparing herbicidally active compositions, which comprises mixing a herbicidally active amount of at least one substituted 2-phenylpyridine of the formula I or of an agriculturally utilizable salt of I, as claimed in claim 1, and at least one inert liquid and/or solid carrier and also, if desired, at least one surface-active substance.

**7.** A process for preparing compositions having desiccant and/or defoliant activity, which comprises mixing an amount of at least one substituted 2-phenylpyridine of the formula I or of an agriculturally utilizable salt of I, as claimed in claim 1, having desiccant and/or defoliant activity, and at least one inert liquid and/or solid carrier and also, if desired, at least one surface-active substance.

**8.** A method of controlling undesired vegetation, which comprises allowing a herbicidally active amount of at least one substituted 2-phenylpyridine of the formula I or of an agriculturally utilizable salt of I, as claimed in claim 1, to act on plants, their habitat or on seed.

**9.** A method for the desiccation and/or defoliation of plants, which comprises allowing an amount of at least one substituted 2-phenylpyridine of the formula I or of an agriculturally utilizable salt of I, as claimed in claim 1, having dessicant and/or defoliant activity, to act on plants.

**10.** A method as claimed in claim 9, wherein cotton is treated.

**11.** A process for preparing substituted 2-phenylpyridines of the formula I as claimed in claim 1, where Z is the group

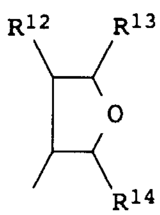

which comprises reacting an acid chloride of the formula III

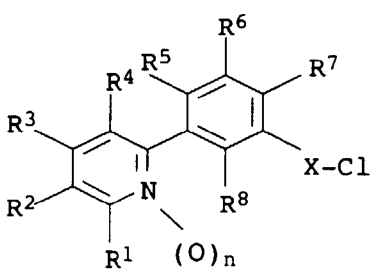

III

in a manner known per se in an inert solvent or diluent, in the presence of a base, with a 3-hydroxytetrahydrofuran of the formula IV

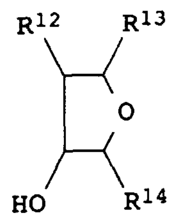

IV.

**12.** A process for preparing substituted 2-phenylpyridines of the formula I as claimed in claim 1, where Z is the group

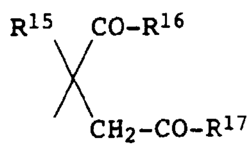

which comprises reacting an acid chloride of the formula III

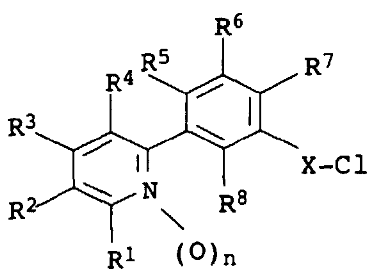

III

in a manner known per se in an inert solvent or diluent, in the presence of a base, with an alcohol of the formula V

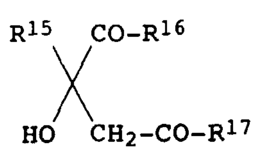

V.

**13.** An acid chloride of the formula III

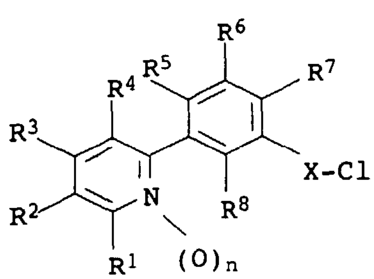

III

where the variables have the following meanings:

| | |
|---|---|
| n | is 0 or 1; |
| $R^1$, $R^3$ and $R^4$ | independently of one another are hydrogen, halogen, $C_1$-$C_4$-alkyl, $C_1$-$C_4$-haloalkyl, hydroxyl, $C_1$-$C_4$-alkoxy, $C_1$-$C_4$-haloalkoxy, nitro, amino, $C_1$-$C_4$-alkylamino, di-($C_1$-$C_4$-alkyl)-amino, mercapto, $C_1$-$C_4$-alkylthio, $C_1$-$C_4$-haloalkylthio, cyano, carboxyl, ($C_1$-$C_4$-alkoxy)carbonyl, aminocarbonyl, $C_1$-$C_4$-alkylaminocarbonyl or di-($C_1$-$C_4$-alkyl)-aminocarbonyl; |
| $R^2$ | is hydrogen, halogen, $C_1$-$C_4$-alkyl, $C_1$-$C_4$-haloalkyl, cyano, nitro, amino, hydroxyl, $C_1$-$C_4$-alkoxy, $C_1$-$C_4$-haloalkoxy, mercapto, $C_1$-$C_4$-alkylthio, $C_1$-$C_4$-haloalkylthio, $C_1$-$C_4$-alkylsulfinyl, $C_1$-$C_4$-haloalkylsulfinyl, $C_1$-$C_4$-alkylsulfonyl or $C_1$-$C_4$-haloalkylsulfonyl; |
| $R^5$ | is hydrogen, halogen or cyano; |
| $R^6$ and $R^8$ | independently of one another are hydrogen or halogen; |
| $R^7$ | is hydrogen, cyano, nitro, hydroxyl, trifluoromethylsulfonyloxy, halogen, $C_1$-$C_4$-alkyl, $C_1$-$C_4$-haloalkyl, $C_1$-$C_4$-alkoxy or $C_1$-$C_4$-haloalkoxy; |
| X | is one of the following bridges: -CO-, -O-C($R^9$,$R^{10}$)-CO-, -S-C($R^9$,$R^{10}$)-CO-, -$CH_2$-CH($R^{11}$)-CO- or -CH=C($R^{11}$)-CO-, where $R^9$ and $R^{10}$ independently of one another are hydrogen or $C_1$-$C_4$-alkyl and $R^{11}$ is hydrogen, halogen or $C_1$-$C_4$-alkyl. |

**14.** A process for preparing the acid chlorides of the formula III as claimed in claim 13, which comprises chlorinating the corresponding free carboxylic acids or their alkali metal salts in a manner known per se.

**15.** A process for preparing substituted 2-phenylpyridines of the formula I as claimed in claim 1, where n is 1, which comprises oxidizing the corresponding substituted 2-phenylpyridines, where n is zero, in a manner known per se in an inert solvent or diluent.

**16.** A process for preparing substituted 2-phenylpyridines of the formula I as claimed in claim 1, where X is a bridge -O-C($R^9$,$R^{10}$)-CO-, which comprises reacting a 3-pyridylphenol of the formula VI

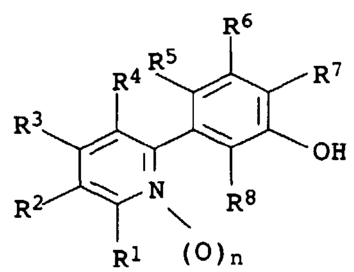

VI

in a manner known per se in an inert solvent or diluent, in the presence of a base, with a tetrahydrofuryl ester of the formula VII

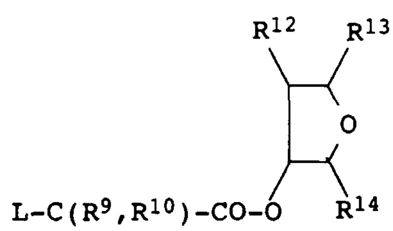

VII

or an ester of the formula VIII

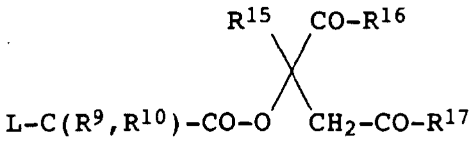

VIII

where L in each case is chlorine, bromine, iodine, methylsulfonyloxy, trifluoromethylsulfonyloxy, phenylsulfonyloxy or p-toluenesulfonyloxy.

**17.** A process for preparing substituted 2-phenylpyridines of the formula I as claimed in claim 1, where X is a bridge -S-C($R^9$,$R^{10}$)-CO-, which comprises reacting a 3-pyridylthiophenol of the formula IX

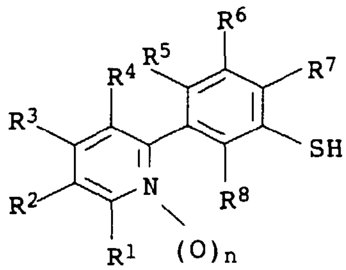

IX

in a manner known per se in an inert solvent or diluent, in the presence of a base, with a tetrahydrofuryl ester of the formula VII

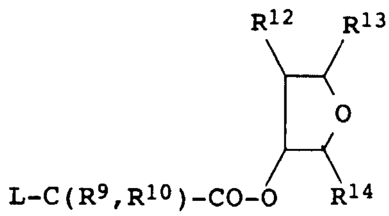

VII

or an ester of the formula VIII

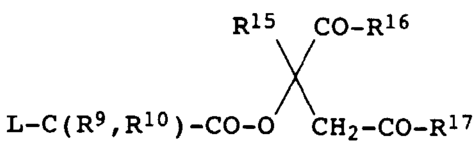

VIII

where L in each case is chlorine, bromine, iodine, methylsulfonyloxy, trifluoromethylsulfonyloxy, phenylsulfonyloxy or p-tolylsulfonyloxy.

**Revendications**

**1.** 2-phénylpyridines substituées de formule générale I

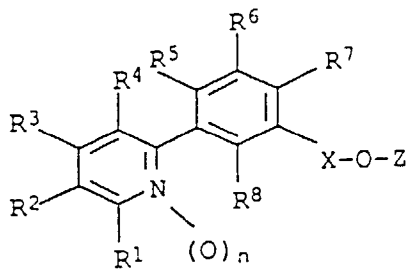

I

dans laquelle les symboles ont les significations suivantes :

n : 0 ou 1 ;

$R^1$, $R^3$, $R^4$, indépendamment les uns des autres, l'hydrogène, un halogène, un groupe alkyle en C1-C4, halogénoalkyle en C1-C4, hydroxy, alcoxy en C1-C4, halogénoalcoxy en C1-C4, nitro, amino, alkylamino en C1-C4, di-(alkyle en C1-C4)amine, mercapto, alkylthio en C1-C4, halogénoalkylthio en C1-C4, cyano, carboxyle, (alcoxy en C1-C4)carbonyle, aminocarbonyle, (alkyle en C1-C4)aminocarbonyle ou di-(alkyle en C1-C4)aminocarbonyle ;

$R^2$ : l'hydrogène, un halogène, un groupe alkyle en C1-C4, halogénoalkyle en C1-C4, cyano, nitro, amino, hydroxy, alcoxy en C1-C4, halogénoalcoxy en C1-C4, mercapto, alkylthio en C1-C4, halogénoalkylthio en C1-C4, alkylsulfinyle en C1-C4, halogénoalkylsulfinyle en C1-C4, alkylsulfonyle en C1-C4 ou halogénoalkylsulfonyle en C1-C4 ;

$R^5$ : l'hydrogène, un halogène ou un groupe cyano ;

$R^6$ et $R^8$, indépendamment l'un de l'autre, l'hydrogène ou un halogène ;

$R^7$ : l'hydrogène, un groupe cyano, nitro, hydroxy, trifluorométhylsulfonyloxy, un halogène, un groupe alkyle en C1-C4, halogénoalkyle en C1-C4, alcoxy en C1-C4 ou halogénoalcoxy en C1-C4;

X : l'un des ponts suivants : -CO-, -O-C($R^9$, $R^{10}$)-CO-, -S-C($R^9$, $R^{10}$)-CO-, $-CH_2-CH(R^{11})-CO-$ ou $-CH=C(R^{11})-CO-$, dans lesquels $R^9$ et $R^{10}$ représentent chacun, indépendamment l'un de l'autre, l'hydrogène ou un groupe alkyle en C1-C4 et $R^{11}$ représente l'hydrogène, un halogène ou un groupe alkyle en C1-C4 ;

Z : représente un groupe

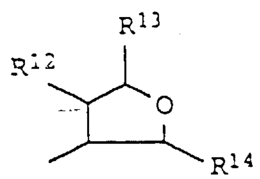

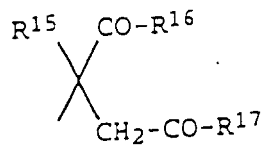

dans lequel

$R^{12}$ représente l'hydrogène, une groupe hydroxy, un halogène, un groupe alkyle en C1-C4, alcoxy en C1-C4, (alkyle en C1-C4)carbonyloxy, (halogénoalkyle en C1-C4)carbonyloxy, (cycloalkyle en C3-C6)carbonyloxy, benzoyloxy, benzylcarbonyloxy, benzyloxy, (alcoxy en C1-C4)carbonyloxy, (alkylamino en C1-C4)carbonyloxy, di-(alkyle en C1-C4)aminocarbonyloxy ou alkylsulfonyloxy en C1-C3 ; $R^{13}$ et $R^{14}$ représentent chacun, indépendemment l'un de l'autre, l'hydrogène, un groupe hydroxy, alkyle en C1-C4, alcoxy en C1-C4 ou (alkyle en C1-C4)carbonyloxy ;

$R^{15}$ représente l'hydrogène ou un groupe alkyle en C1-C4 et $R^{16}$ et $R^{17}$ représentent chacun, indépendamment l'un de l'autre, un groupe hydroxy, alcoxy en C1-C6, alcényloxy en C3-C6, alcynyloxy en C3-C6, (alcoxy en Cl-C6)alcoxy en C2-C4, cycloalcoxy en C3-C6, amino, alkylamino en C1-C4, di-(alkyle en C1-C4)amine, N-pyrrolidinyle, N-pipéridinyle, N-morpholinyle, cycloalkylamino en C3-C6, di(cycloalkyle en C3-C6)amino, alcénylamino en C3-C6 ou alcynylamino en C3-C6, ou bien

$R^{16}$ et $R^{17}$ forment ensemble un pont -N($R^{18}$)-N($R^{19}$)-ou-N($R^{20}$)-, dans lesquels

$R^{18}$ et $R^{19}$ représentent chacun l'hydrogène ou un groupe alkyle en C1-C4 et $R^{20}$ représente l'hydrogène, un groupe alkyle en C1-C6, alcényle en C3-C6, alcynyle en C3-C6, alcoxy en C1-C6 ou alcényloxy en C3-C6, et les sels des composés I acceptables pour des usages agricoles, lorsqu'ils existent.

**2.** 2-phénylpyridines substitués de formule I selon la revendication 1 dans laquelle n est égal à 0, $R^1$, $R^3$, $R^4$ représentent chacun, indépendamment les uns des autres, l'hydrogène ou un halogène, $R^2$ représente un halogène ou un groupe halogénoalkyle en $C_1$-$C_4$ contenant un à cinq atomes d'halogènes, $R^5$ représente l'hydrogène ou un halogène, $R^6$ représente l'hydrogène, $R^7$ un halogène ou un groupe cyano, $R^8$ l'hydrogène, $R^9$ et $R^{10}$, indépendamment l'un de l'autre, l'hydrogène ou un groupe alkyle en C1-C4, $R^{11}$ représente l'hydrogène ou un halogène, $R^{12}$, $R^{13}$, $R^{14}$ représentent chacun, indépendamment les uns des autres, l'hydrogène, un groupe alkyle en C1-C4, hydroxy ou (alkyle en C1-C4)carbonyloxy, $R^{15}$ représente l'hydrogène, $R^{16}$ et $R^{17}$ représentent chacun, indépendamment l'un de l'autre, un groupe alcoxy en C1-C6, alcényloxy en C3-C6, amino, alkylamino en C1-C4, di-(alkyle en C1-C4)amino ou forment ensemble un pont -NH-NH-, -N(alkyle en C1-C4)-N-(alkyle en C1-C4)-ou-N($R^{20}$)- et $R^{20}$ représente l'hydrogène ou un groupe alkyle en C1-C6.

**3.** Utilisation des 2-phénylpyridines substituées de formule I et de leurs sels acceptables pour des applications agricoles, selon la revendication 1, en tant qu'herbicides ou pour la dessiccation et/ou la défoliation des végétaux.

**4.** Produit herbicide contenant une quantité herbicide efficace d'au moins une 2-phénylpyridine substituée de formule I ou de l'un de ses sels acceptables pour les usages agricoles, selon la revendication 1, et au moins un véhicule solide et/ou liquide inerte, et si on le désire, au moins une substance tensio-active.

**5.** Produit pour la dessication et/ou la défoliation des végétaux, contenant une quantité dessiccante et/ou défoliante efficace d'au moins une 2-phénylpyridine substituée de formule I ou de l'un de ses sels acceptables pour les usages agricoles, selon la revendication 1, et au moins un véhicule solide et/ou liquide inerte, ainsi que, si on le désire, au moins une substance tensio-active.

**6.** Procédé de préparation de produits herbicides, caractérisé par le fait que l'on mélange une quantité herbicide efficace d'au moins une 2-phénylpyridine substituée de formule I ou de l'un de ses sels acceptables pour les usages agricoles, selon la revendication 1, avec au moins un véhicule solide et/ou liquide inerte et, si on le désire, au moins une substance tensio-active.

**7.** Procédé de préparation de produits dessicants et/ou défoliants, caractérisé par le fait que l'on mélange une quantité dessicante et/ou défoliante efficace d'au moins une 2-phénylpyridine substituée de formule I ou de l'un de ses sels acceptables pour les applications agricoles, selon la revendication 1, avec au moins un véhicule solide et/ou liquide inerte et, si on le désire, au moins une substance tensio-active.

**8.** Procédé pour combattre les croissances des végétaux indésirables, caractérisé par le fait que l'on fait agir une quantité herbicide efficace d'au moins une 2-phényl-pyridine substituée de formule I ou de l'un de ses sels convenant pour les applications agricoles, selon la revendication 1, sur les végétaux, leur habitat ou leurs semences.

**9.** Procédé pour la dessication et/ou la défoliation de végétaux, caractérisé par le fait que l'on fait agir une quantité dessicante et/ou défoliante efficace d'au moins une 2-phénylpyridine substituée de formule I ou de l'un de ses sels convenant pour les applications agricoles, selon la revendication 1, sur les végétaux.

**10.** Procédé selon la revendication 9, caractérisé par le fait que l'on traite le coton.

**11.** Procédé pour la préparation des 2-phénylpyridines substituées de formule I de la revendication 1, dans laquelle Z représente le groupe

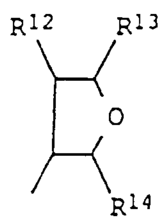

caractérisé par le fait que l'on fait réagir un chlorure d'acide de formule III

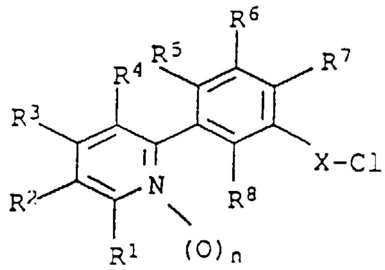

III

de manière connue en soi, dans un solvant ou diluant inerte et en présence d'une base, avec un 3-hydroxytétrahydrofuranne de formule IV

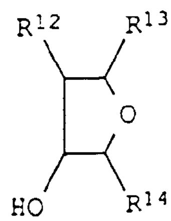

IV

**12.** Procédé de préparation des 2-phénylpyridines substituées de formule I de la revendication 1, dans laquelle Z représente le groupe

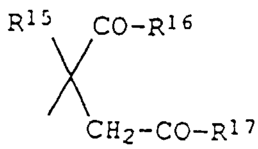

caractérisé par le fait que l'on fait réagir un chlorure d'acide de formule III.

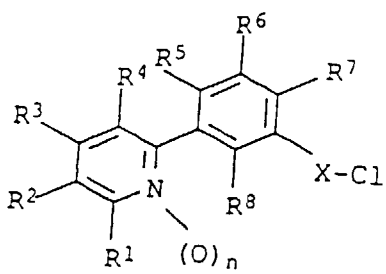

III

de manière connue en soi, dans un solvant ou diluant inerte et e présence d'une base, avec un alcool de formule V

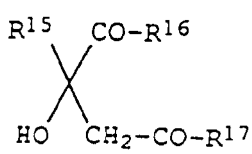

V

**13.** Chlorures d'acides de formule III

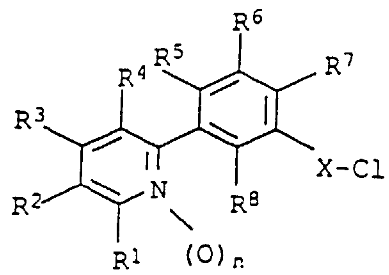

III

dans laquelle les symboles ont les significations suivantes :

n: 0 ou 1 ;
$R^1$, $R^3$, $R^4$ : indépendamment les uns des autres :
l'hydrogène, un halogène, un groupe alkyle en C1-C4, halogénoalkyle en C1-C4, hydroxy, alcoxy en C1-C4, halogénoalcoxy en C1-C4, nitro, amino, alkylamino en C1-C4, di-(alkyle en C1-C4)amine, mercapto, alkylthio en C1-C4, halogénoalkylthio en C1-C4, cyano, carboxyle, (alcoxy en C1-C4)carbonyle, aminocarbonyle, (alkyle en C1-C4)aminocarbonyle ou di-(alkyle en C1-C4)aminocarbonyle ;
$R^2$ : l'hydrogène, un halogène, un groupe alkyle en C1-C4, halogénoalkyle en C1-C4, cyano, nitro, amino, hydroxy, alcoxy en C1-C4, halogénoalcoxy en C1-C4, mercapto, alkylthio en C1-C4, halogénoalkylthio en C1-C4, alkylsulfinyle en C1-C4, halogénoalkylsulfinyle en C1-C4, alkylsulfonyle en C1-C4 ou halogénoalkylsulfonyle en C1-C4 ;
$R^5$ : l'hydrogène, un halogène ou un groupe cyano ;
$R^6$ et $R^8$, indépendamment l'un de l'autre, l'hydrogène ou un halogène ;
$R^7$ : l'hydrogène, une groupe cyano, nitro, hydroxy, trifluorométhylsulfonyloxy, un halogène, un groupe alkyle en C1-C4, halogénoalkyle en C1-C4, alcoxy en C1-C4 ou halogéno-alcoxy en C1-C4 ;
X : l'un des ponts suivants :
-CO-, -O-C($R^9$, $R^{10}$)-CO-, -S-C($R^9$, $R^{10}$)-CO-, -CH2-CH($R^{11}$)-CO- ou-CH=C($R^{11}$)-CO-, dans lesquels $R^9$ et $R^{10}$ représentent chacun, indépendamment l'un de l'autre, l'hydrogène ou un groupe alkyle en C1-C4 et $R^{11}$ représente l'hydrogène, un halogène ou un groupe alkyle en C1-C4.

**14.** Procédé de préparation des chlorures d'acides de formule III de la revendication 13, caractérisé par le fait que l'on chlore de manière connue en soi les acides carboxyliques libres correspondants ou leurs sels de métaux alcalins.

**15.** Procédé de préparation des 2-phénylpyridines substituées de formule I selon la revendication 1 dans laquelle n est égal à 1, caractérisé par le fait que l'on oxyde de manière connue en soi, dans un solvant ou diluant inerte, les 2-phénylpyridines substituées correspondantes pour lesquelles n est égal à 0.

**16.** Procédé de préparation des 2-phénylpyridines substituées de formule I de la revendication 1 dans laquelle X représente une pont -O-C($R^9$, $R^{10}$)-CO-, caractérisé par le fait que l'on fait réagir un 3-pyridylphénol de formule VI.

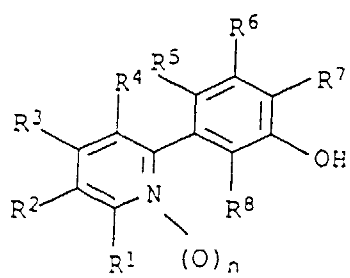

VI

de manière connue en soi, dans un solvant ou diluant inerte et en présence d'une base, avec un ester tétrahydrofurylique de formule VII

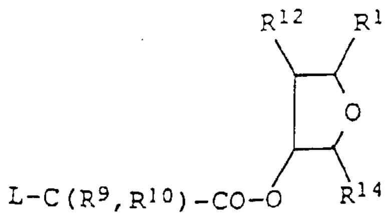

VII

ou un ester de formule VIII

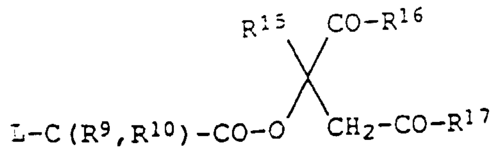

VIII

dans lesquelles L représente le chlore, le brome, l'iode, un groupe méthylsulfonyloxy, trifluorométhylsulfonyloxy, phénylsulfonyloxy ou p-toluènesulfonyloxy.

**17.** Procédé de préparation des 2-phénylpyridines subsitituées de formule I de la revendication 1 dans laquelle X représente un pont -S-C($R^9R^{10}$)-CO-, caractérisé par le fait que l'on fait réagir un 3-pyridylthiophénol de formule IX

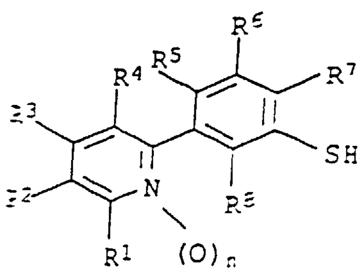

IX

de manière connue en soi, dans un solvant ou diluant inerte en présence d'une base, avec un ester tétrahydrofurylique de formule VII

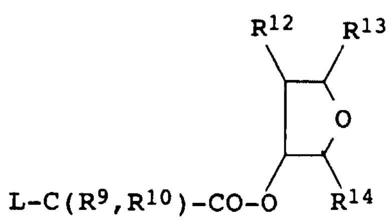

VII

ou avec un ester de formule VIII

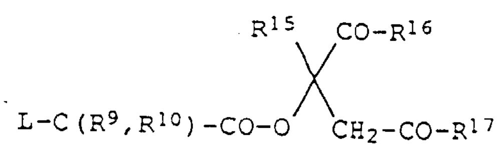

VIII

dans lesquelles L représente le chlore, le brome, l'iode, un groupe méthylsulfonyloxy, trifluorométhylsulfonyloxy, phénylsulfonyloxy ou p-tolylsulfonyloxy.